# EUROPEAN PATENT SPECIFICATION

(11) **EP 1 140 136 B1**
(45) Date of publication and mention of the grant of the patent: **16.05.2007**
(21) Application number: 00955737.2
(22) Date of filing: 18.08.2000
(51) Int. Cl.: A61K 38/17, A61K 39/395, A61P 19/00, A61P 35/02, A61P 35/04

(54) **COMPOSITIONS AND METHODS FOR THE PREVENTION OR TREATMENT OF CANCER AND BONE LOSS ASSOCIATED WITH CANCER**
VERFAHREN UND ZUSAMMENSETZUNGEN ZUR PREVENTION ODER BEHANDLUNG VON KREBS UND VON KREBS ASSOZIERTEM KNOCHENVERLUST
COMPOSITIONS ET PROCEDES PERMETTANT LA PREVENTION OU LE TRAITEMENT DU CANCER ET DE LA PERTE OSSEUSE ASSOCIEE AU CANCER

(30) Priority: 03.09.1999 US 389545
(43) Date of publication of application: 10.10.2001
(60) Divisional application: 06022155.3
(73) Proprietor: AMGEN INC., Thousand Oaks, CA 91320-1799 (US)
(72) Inventor: DUNSTAN, Colin, R., Newbury Park, CA 91320 (US)
(74) Representative: Grünecker, Kinkeldey, Stockmair & Schwanhäusser Anwaltssozietät
(86) International application number: PCT/US2000/022806
(87) International publication number: WO 2001/017543

(56) References cited:
- EP-A- 0 784 093
- US-A- 5 843 901
- SIMONET W ET AL: "Osteoprotegerin: a novel secreted protein involved in the regulation of bone density" CELL,US,CELL PRESS, CAMBRIDGE, NA, no. 89, 18 April 1997 (1997-04-18), pages 309-319, XP002077048 ISSN: 0092-8674
- MORONY S ET AL: "A CHIMERIC FORM OF OSTEOPROTEGERIN INHIBITS HYPERCALCEMIA AND BONE RESORPTION INDUCED BY IL-1BETA, TNF-ALPHA, PTH, PTHRP, AND 1,25(OH)2D3" JOURNAL OF BONE AND MINERAL RESEARCH,NEW YORK, NY,US, vol. 14, no. 9, September 1999 (1999-09), pages 1478-1485, XP000971493 ISSN: 0884-0431
- PERIGAUD C ET AL: "NUCLEOSIDE ANALOGUES AS CHEMOTHERAPEUTIC AGENTS: A REVIEW" NUCLEOSIDES & NUCLEOTIDES,US,DEKKER, NEW YORK,NY,, vol. 11, no. 2/04, 1992, pages 903-945, XP000607988 ISSN: 0732-8311
- X YANG ET AL: "Eradication of established tumors by a fully human monoclonal antibody to the epidermal growth factor receptor without concomitant chemotherapy" CANCER RESEARCH,AMERICAN ASSOCIATION FOR CANCER RESEARCH, BALTIMORE, MD,US, vol. 59, no. 5, 15 March 1999 (1999-03-15), pages 1236-1243, XP002156618 ISSN: 0008-5472

### SEQUENCE LISTING

<110> AMGEN INC.
<120> COMPOSITIONS AND METHODS FOR THE PREVENTION OR TREATMENT OF CANCER AND BONE LOSS ASSOCIATED WITH CANCER
<130> A-605
<140> 09/389,545
   <141> 1999-09-03
<160> 8
<170> PatentIn Ver. 2.1
<210> 1
   <211> 232
   <212> PRT
   <213> Human
<400> 1
   Glu Pro Lys Ser Cys Asp Lys Thr His Thr Cys Pro Pro Cys Pro Ala
    1               5                   10                  15
   Pro Glu Leu Leu Gly Gly Pro Ser Val Phe Leu Phe Pro Pro Lys Pro
               20                  25                  30
   Lys Asp Thr Leu Met Ile Ser Arg Thr Pro Glu Val Thr Cys Val Val
           35                  40                  45
   Val Asp Val Ser His Glu Asp Pro Glu Val Lys Phe Asn Trp Tyr Val
       50                  55                  60
   Asp Gly Val Glu Val His Asn Ala Lys Thr Lys Pro Arg Glu Glu Gln
   65                  70                  75                  80
   Tyr Asn Ser Thr Tyr Arg Val Val Ser Val Leu Thr Val Leu His Gln
                   85                  90                  95
   Asp Trp Leu Asn Gly Lys Glu Tyr Lys Cys Lys Val Ser Asn Lys Ala
               100                 105                 110
   Leu Pro Ala Pro Ile Glu Lys Thr Ile Ser Lys Ala Lys Gly Gln Pro
           115                 120                 125
   Arg Glu Pro Gln Val Tyr Thr Leu Pro Pro Ser Arg Asp Glu Leu Thr
       130                 135                 140
   Lys Asn Gln Val Ser Leu Thr Cys Leu Val Lys Gly Phe Tyr Pro Ser
   145                 150                 155                 160
   Asp Ile Ala Val Glu Trp Glu Ser Asn Gly Gln Pro Glu Asn Asn Tyr
                   165                 170                 175
   Lys Thr Thr Pro Pro Val Leu Asp Ser Asp Gly Ser Phe Phe Leu Tyr
               180                 185                 190
   Ser Lys Leu Thr Val Asp Lys Ser Arg Trp Gln Gln Gly Asn Val Phe
           195                 200                 205
   Ser Cys Ser Val Met His Glu Ala Leu His Asn His Tyr Thr Gln Lys
       210                 215                 220
   Ser Leu Ser Leu Ser Pro Gly Lys
   225                 230
<210> 2
   <211> 401
   <212> PRT
   <213> Human
<400> 2
   Met Asn Lys Trp Leu Cys Cys Ala Leu Leu Val Leu Leu Asp Ile Ile
    1               5                  10                  15
   Glu Trp Thr Thr Gln Glu Thr Leu Pro Pro Lys Tyr Leu His Tyr Asp
               20                  25                  30
   Pro Glu Thr Gly His Gln Leu Leu Cys Asp Lys Cys Ala Pro Gly Thr
           35                  40                  45
   Tyr Leu Lys Gln His Cys Thr Val Arg Arg Lys Thr Leu Cys Val Pro
       50                  55                  60
   Cys Pro Asp His Ser Tyr Thr Asp Ser Trp His Thr Ser Asp Glu Cys
   65                  70                  75                  80
   Val Tyr Cys Ser Pro Val Cys Lys Glu Leu Gln Ser Val Lys Gln Glu
                   85                  90                  95
   Cys Asn Arg Thr His Asn Arg Val Cys Glu Cys Glu Glu Gly Arg Tyr
               100                 105                 110
   Leu Glu Ile Glu Phe Cys Leu Lys His Arg Ser Cys Pro Pro Gly Ser
           115                 120                 125
   Gly Val Val Gln Ala Gly Thr Pro Glu Arg Asn Thr Val Cys Lys Lys
       130                 135                 140
   Cys Pro Asp Gly Phe Phe Ser Gly Glu Thr Ser Ser Lys Ala Pro Cys
   145                 150                 155                 160
   Ile Lys His Thr Asn Cys Ser Thr Phe Gly Leu Leu Leu Ile Gln Lys
                   165                 170                 175
   Gly Asn Ala Thr His Asp Asn Val Cys Ser Gly Asn Arg Glu Ala Thr
               180                 185                 190
   Gln Lys Cys Gly Ile Asp Val Thr Leu Cys Glu Glu Ala Phe Phe Arg
           195                 200                 205
   Phe Ala Val Pro Thr Lys Ile Ile Pro Asn Trp Leu Ser Val Leu Val
       210                 215                 220
   Asp Ser Leu Pro Gly Thr Lys Val Asn Ala Glu Ser Val Glu Arg Ile
   225                 230                 235                 240
   Lys Arg Arg His Ser Ser Gln Glu Gln Thr Phe Gln Leu Leu Lys Leu
                   245                 250                 255
   Trp Lys His Gln Asn Arg Asp Gln Glu Met Val Lys Lys Ile Ile Gln
   260 265 270
   Asp Ile Asp Leu Cys Glu Ser Ser Val Gln Arg His Leu Gly His Ser
   275 280 285
   Asn Leu Thr Thr Glu Gln Leu Leu Ala Leu Met Glu Ser Leu Pro Gly
   290 295 300
   Lys Lys Ile Ser Pro Glu Glu Ile Glu Arg Thr Arg Lys Thr Cys Lys
   305 310 315 320
   Ser Ser Glu Gln Leu Leu Lys Leu Leu Ser Leu Trp Arg Ile Lys Asn
   325 330 335
   Gly Asp Gln Asp Thr Leu Lys Gly Leu Met Tyr Ala Leu Lys His Leu
   340 345 350
   Lys Thr Ser His Phe Pro Lys Thr Val Thr His Ser Leu Arg Lys Thr
   355 360 365
   Met Arg Phe Leu His Ser Phe Thr Met Tyr Arg Leu Tyr Gln Lys Leu
   370 375 380
   Phe Leu Glu Met Ile Gly Asn Gln Val Gln Ser Val Lys Ile Ser Cys
   385 390 395 400
   Leu
<210> 3
   <211> 407
   <212> PRT
   <213> Human
<400> 3
   Glu Thr Phe Pro Pro Lys Tyr Leu His Tyr Asp Glu Glu Thr Ser His
   1 5 10 15
   Gln Leu Leu Cys Asp Lys Cys Pro Pro Gly Thr Tyr Leu Lys Gln His
   20 25 30
   Cys Thr Ala Lys Trp Lys Thr Val Cys Ala Pro Cys Pro Asp His Tyr
   35 40 45
   Tyr Thr Asp Ser Trp His Thr Ser Asp Glu Cys Leu Tyr Cys Ser Pro
   50 55 60
   Val Cys Lys Glu Leu Gln Tyr Val Lys Gln Glu Cys Asn Arg Thr His
   65 70 75 80
   Asn Arg Val Cys Glu Cys Lys Glu Gly Arg Tyr Leu Glu Ile Glu Phe
   85 90 95
   Cys Leu Lys His Arg Ser Cys Pro Pro Gly Phe Gly Val Val Gln Ala
   100 105 110
   Gly Thr Pro Glu Arg Asn Thr Val Cys Lys Arg Cys Pro Asp Gly Phe
   115 120 125
   Phe Ser Asn Glu Thr Ser Ser Lys Ala Pro Cys Arg Lys His Thr Asn
       130                 135                 140
   Cys Ser Val Phe Gly Leu Leu Leu Thr Gln Lys Gly Asn Ala Thr His
   145                 150                 155                 160
   Asp Asn Ile Cys Ser Gly Asn Ser Glu Ser Thr Gln Lys Ala Ala Ala
                   165                 170                 175
   Glu Pro Lys Ser Cys Asp Lys Thr His Thr Cys Pro Pro Cys Pro Ala
               180                 185                 190
   Pro Glu Leu Leu Gly Gly Pro Ser Val Phe Leu Phe Pro Pro Lys Pro
           195                 200                 205
   Lys Asp Thr Leu Met Ile Ser Arg Thr Pro Glu Val Thr Cys Val Val
       210                 215                 220
   Val Asp Val Ser His Glu Asp Pro Glu Val Lys Phe Asn Trp Tyr Val
   225                 230                 235                 240
   Asp Gly Val Glu Val His Asn Ala Lys Thr Lys Pro Arg Glu Glu Gln
                   245                 250                 255
   Tyr Asn Ser Thr Tyr Arg Val Val Ser Val Leu Thr Val Leu His Gln
               260                 265                 270
   Asp Trp Leu Asn Gly Lys Glu Tyr Lys Cys Lys Val Ser Asn Lys Ala
           275                 280                 285
   Leu Pro Ala Pro Ile Glu Lys Thr Ile Ser Lys Ala Lys Gly Gln Pro
       290                 295                 300
   Arg Glu Pro Gln Val Tyr Thr Leu Pro Pro Ser Arg Asp Glu Leu Thr
   305                 310                 315                 320
   Lys Asn Gln Val Ser Leu Thr Cys Leu Val Lys Gly Phe Tyr Pro Ser
                   325                 330                 335
   Asp Ile Ala Val Glu Trp Glu Ser Asn Gly Gln Pro Glu Asn Asn Tyr
               340                 345                 350
   Lys Thr Thr Pro Pro Val Leu Asp Ser Asp Gly Ser Phe Phe Leu Tyr
           355                 360                 365
   Ser Lys Leu Thr Val Asp Lys Ser Arg Trp Gln Gln Gly Asn Val Phe
       370                 375                 380
   Ser Cys Ser Val Met His Glu Ala Leu His Asn His Tyr Thr Gln Lys
   385                 390                 395                 400
   Ser Leu Ser Leu Ser Pro Gly
                   405
<210> 4
   <211> 413
   <212> PRT
   <213> Human
<400> 4
   Glu Thr Phe Pro Pro Lys Tyr Leu His Tyr Asp Glu Glu Thr Ser His
   1 5 10 15
   Gln Leu Leu Cys Asp Lys Cys Pro Pro Gly Thr Tyr Leu Lys Gln His
   20 25 30
   Cys Thr Ala Lys Trp Lys Thr Val Cys Ala Pro Cys Pro Asp His Tyr
   35 40 45
   Tyr Thr Asp Ser Trp His Thr Ser Asp Glu Cys Leu Tyr Cys Ser Pro
   50 55 60
   Val Cys Lys Glu Leu Gln Tyr Val Lys Gln Glu Cys Asn Arg Thr His
   65 70 75 80
   Asn Arg Val Cys Glu Cys Lys Glu Gly Arg Tyr Leu Glu Ile Glu Phe
   85 90 95
   Cys Leu Lys His Arg Ser Cys Pro Pro Gly Phe Gly Val Val Gln Ala
   100 105 110
   Gly Thr Pro Glu Arg Asn Thr Val Cys Lys Arg Cys Pro Asp Gly Phe
   115 120 125
   Phe Ser Asn Glu Thr Ser Ser Lys Ala Pro Cys Arg Lys His Thr Asn
   130 135 140
   Cys Ser Val Phe Gly Leu Leu Leu Thr Gln Lys Gly Asn Ala Thr His
   145 150 155 160
   Asp Asn Ile Cys Ser Gly Asn Ser Glu Ser Thr Gln Lys Cys Gly Ile
   165 170 175
   Asp Val Thr Ala Ala Ala Glu Pro Lys Ser Cys Asp Lys Thr His Thr
   180 185 190
   Cys Pro Pro Cys Pro Ala Pro Glu Leu Leu Gly Gly Pro Ser Val Phe
   195 200 205
   Leu Phe Pro Pro Lys Pro Lys Asp Thr Leu Met Ile Ser Arg Thr Pro
   210 215 220
   Glu Val Thr Cys Val Val Val Asp Val Ser His Glu Asp Pro Glu Val
   225 230 235 240
   Lys Phe Asn Trp Tyr Val Asp Gly Val Glu Val His Asn Ala Lys Thr
   245 250 255
   Lys Pro Arg Glu Glu Gln Tyr Asn Ser Thr Tyr Arg Val Val Ser Val
   260 265 270
   Leu Thr Val Leu His Gln Asp Trp Leu Asn Gly Lys Glu Tyr Lys Cys
   275 280 285
   Lys Val Ser Asn Lys Ala Leu Pro Ala Pro Ile Glu Lys Thr Ile Ser
   290 295 300
   Lys Ala Lys Gly Gln Pro Arg Glu Pro Gln Val Tyr Thr Leu Pro Pro
   305 310 315 320
   Ser Arg Asp Glu Leu Thr Lys Asn Gln Val Ser Leu Thr Cys Leu Val
   325 330 335
   Lys Gly Phe Tyr Pro Ser Asp Ile Ala Val Glu Trp Glu Ser Asn Gly
   340 345 350
   Gln Pro Glu Asn Asn Tyr Lys Thr Thr Pro Pro Val Leu Asp Ser Asp
   355 360 365
   Gly Ser Phe Phe Leu Tyr Ser Lys Leu Thr Val Asp Lys Ser Arg Trp
   370 375 380
   Gln Gln Gly Asn Val Phe Ser Cys Ser Val Met His Glu Ala Leu His
   385 390 395 400
   Asn His Tyr Thr Gln Lys Ser Leu Ser Leu Ser Pro Gly
   405 410
<210> 5
   <211> 400
   <212> PRT
   <213> Human
<400> 5
   Glu Thr Phe Pro Pro Lys Tyr Leu His Tyr Asp Glu Glu Thr Ser His
    1               5                  10                  15
   Gln Leu Leu Cys Asp Lys Cys Pro Pro Gly Thr Tyr Leu Lys Gln His
               20                  25                  30
   Cys Thr Ala Lys Trp Lys Thr Val Cys Ala Pro Cys Pro Asp His Tyr
           35                  40                  45
   Tyr Thr Asp Ser Trp His Thr Ser Asp Glu Cys Leu Tyr Cys Ser Pro
       50                  55                  60
   Val Cys Lys Glu Leu Gln Tyr Val Lys Gln Glu Cys Asn Arg Thr His
   65                  70                  75                  80
   Asn Arg Val Cys Glu Cys Lys Glu Gly Arg Tyr Leu Glu Ile Glu Phe
                   85                  90                  95
   Cys Leu Lys His Arg Ser Cys Pro Pro Gly Phe Gly Val Val Gln Ala
               100                 105                 110
   Gly Thr Pro Glu Arg Asn Thr Val Cys Lys Arg Cys Pro Asp Gly Phe
           115                 120                 125
   Phe Ser Asn Glu Thr Ser Ser Lys Ala Pro Cys Arg Lys His Thr Asn
       130                 135                 140
   Cys Ser Val Phe Gly Leu Leu Leu Thr Gln Lys Gly Asn Ala Thr His
   145                 150                 155                 160
   Asp Asn Ile Cys Ser Gly Asn Ser Glu Ser Thr Gln Lys Val Asp Lys
                   165                 170                 175
   Thr His Thr Cys Pro Pro Cys Pro Ala Pro Glu Leu Leu Gly Gly Pro
               180                 185                 190
   Ser Val Phe Leu Phe Pro Pro Lys Pro Lys Asp Thr Leu Met Ile Ser
   195 200 205
   Arg Thr Pro Glu Val Thr Cys Val Val Val Asp Val Ser His Glu Asp
   210 215 220
   Pro Glu Val Lys Phe Asn Trp Tyr Val Asp Gly Val Glu Val His Asn
   225 230 235 240
   Ala Lys Thr Lys Pro Arg Glu Glu Gln Tyr Asn Ser Thr Tyr Arg Val
   245 250 255
   Val Ser Val Leu Thr Val Leu His Gln Asp Trp Leu Asn Gly Lys Glu
   260 265 270
   Tyr Lys Cys Lys Val Ser Asn Lys Ala Leu Pro Ala Pro Ile Glu Lys
   275 280 285
   Thr Ile Ser Lys Ala Lys Gly Gln Pro Arg Glu Pro Gln Val Tyr Thr
   290 295 300
   Leu Pro Pro Ser Arg Asp Glu Leu Thr Lys Asn Gln Val Ser Leu Thr
   305 310 315 320
   Cys Leu Val Lys Gly Phe Tyr Pro Ser Asp Ile Ala Val Glu Trp Glu
   325 330 335
   Ser Asn Gly Gln Pro Glu Asn Asn Tyr Lys Thr Thr Pro Pro Val Leu
   340 345 350
   Asp Ser Asp Gly Ser Phe Phe Leu Tyr Ser Lys Leu Thr Val Asp Lys
   355 360 365
   Ser Arg Trp Gln Gln Gly Asn Val Phe Ser Cys Ser Val Met His Glu
   370 375 380
   Ala Leu His Asn His Tyr Thr Gln Lys Ser Leu Ser Leu Ser Pro Gly
   385 390 395 400
<210> 6
   <211> 406
   <212> PRT
   <213> Human
<400> 6
   Glu Thr Phe Pro Pro Lys Tyr Leu His Tyr Asp Glu Glu Thr Ser His
    1               5                  10                  15
   Gln Leu Leu Cys Asp Lys Cys Pro Pro Gly Thr Tyr Leu Lys Gln His
               20                  25                  30
   Cys Thr Ala Lys Trp Lys Thr Val Cys Ala Pro Cys Pro Asp His Tyr
           35                  40                  45
   Tyr Thr Asp Ser Trp His Thr Ser Asp Glu Cys Leu Tyr Cys Ser Pro
       50                  55                  60
   Val Cys Lys Glu Leu Gln Tyr Val Lys Gln Glu Cys Asn Arg Thr His
   65 70 75 80
   Asn Arg Val Cys Glu Cys Lys Glu Gly Arg Tyr Leu Glu Ile Glu Phe
   85 90 95
   Cys Leu Lys His Arg Ser Cys Pro Pro Gly Phe Gly Val Val Gln Ala
   100 105 110
   Gly Thr Pro Glu Arg Asn Thr Val Cys Lys Arg Cys Pro Asp Gly Phe
   115 120 125
   Phe Ser Asn Glu Thr Ser Ser Lys Ala Pro Cys Arg Lys His Thr Asn
   130 135 140
   Cys Ser Val Phe Gly Leu Leu Leu Thr Gln Lys Gly Asn Ala Thr His
   145 150 155 160
   Asp Asn Ile Cys Ser Gly Asn Ser Glu Ser Thr Gln Lys Cys Gly Ile
   165 170 175
   Asp Val Thr Val Asp Lys Thr His Thr Cys Pro Pro Cys Pro Ala Pro
   180 185 190
   Glu Leu Leu Gly Gly Pro Ser Val Phe Leu Phe Pro Pro Lys Pro Lys
   195 200 205
   Asp Thr Leu Met Ile Ser Arg Thr Pro Glu Val Thr Cys Val Val Val
   210 215 220
   Asp Val Ser His Glu Asp Pro Glu Val Lys Phe Asn Trp Tyr Val Asp
   225 230 235 240
   Gly Val Glu Val His Asn Ala Lys Thr Lys Pro Arg Glu Glu Gln Tyr
   245 250 255
   Asn Ser Thr Tyr Arg Val Val Ser Val Leu Thr Val Leu His Gln Asp
   260 265 270
   Trp Leu Asn Gly Lys Glu Tyr Lys Cys Lys Val Ser Asn Lys Ala Leu
   275 280 285
   Pro Ala Pro Ile Glu Lys Thr Ile Ser Lys Ala Lys Gly Gln Pro Arg
   290 295 300
   Glu Pro Gln Val Tyr Thr Leu Pro Pro Ser Arg Asp Glu Leu Thr Lys
   305 310 315 320
   Asn Gln Val Ser Leu Thr Cys Leu Val Lys Gly Phe Tyr Pro Ser Asp
   325 330 335
   Ile Ala Val Glu Trp Glu Ser Asn Gly Gln Pro Glu Asn Asn Tyr Lys
   340 345 350
   Thr Thr Pro Pro Val Leu Asp Ser Asp Gly Ser Phe Phe Leu Tyr Ser
   355 360 365
   Lys Leu Thr Val Asp Lys Ser Arg Trp Gln Gln Gly Asn Val Phe Ser
   370 375 380
   Cys Ser Val Met His Glu Ala Leu His Asn His Tyr Thr Gln Lys Ser
   385 390 395 400
   Leu Ser Leu Ser Pro Gly
   405
<210> 7
   <211> 404
   <212> PRT
   <213> Human
<400> 7
   Glu Thr Phe Pro Pro Lys Tyr Leu His Tyr Asp Glu Glu Thr Ser His
    1               5                  10                  15
   Gln Leu Leu Cys Asp Lys Cys Pro Pro Gly Thr Tyr Leu Lys Gln His
               20                  25                  30
   Cys Thr Ala Lys Trp Lys Thr Val Cys Ala Pro Cys Pro Asp His Tyr
           35                  40                  45
   Tyr Thr Asp Ser Trp His Thr Ser Asp Glu Cys Leu Tyr Cys Ser Pro
       50                  55                  60
   Val Cys Lys Glu Leu Gln Tyr Val Lys Gln Glu Cys Asn Arg Thr His
   65                  70                  75                  80
   Asn Arg Val Cys Glu Cys Lys Glu Gly Arg Tyr Leu Glu Ile Glu Phe
                   85                  90                  95
   Cys Leu Lys His Arg Ser Cys Pro Pro Gly Phe Gly Val Val Gln Ala
               100                 105                 110
   Gly Thr Pro Glu Arg Asn Thr Val Cys Lys Arg Cys Pro Asp Gly Phe
           115                 120                 125
   Phe Ser Asn Glu Thr Ser Ser Lys Ala Pro Cys Arg Lys His Thr Asn
       130                 135                 140
   Cys Ser Val Phe Gly Leu Leu Leu Thr Gln Lys Gly Asn Ala Thr His
   145                 150                 155                 160
   Asp Asn Ile Cys Ser Gly Asn Ser Glu Ser Thr Gln Lys Ser Gly Gly
                   165                 170                 175
   Gly Gly Gly Gly Gly Gly Thr Cys Pro Pro Cys Pro Ala Pro Glu Leu
               180                 185                 190
   Leu Gly Gly Pro Ser Val Phe Leu Phe Pro Pro Lys Pro Lys Asp Thr
           195                 200                 205
   Leu Met Ile Ser Arg Thr Pro Glu Val Thr Cys Val Val Val Asp Val
       210                 215                 220
   Ser His Glu Asp Pro Glu Val Lys Phe Asn Trp Tyr Val Asp Gly Val
   225                 230                 235                 240
   Glu Val His Asn Ala Lys Thr Lys Pro Arg Glu Glu Gln Tyr Asn Ser
                   245                 250                 255
   Thr Tyr Arg Val Val Ser Val Leu Thr Val Leu His Gln Asp Trp Leu
   260 265 270
   Asn Gly Lys Glu Tyr Lys Cys Lys Val Ser Asn Lys Ala Leu Pro Ala
   275 280 285
   Pro Ile Glu Lys Thr Ile Ser Lys Ala Lys Gly Gln Pro Arg Glu Pro
   290 295 300
   Gln Val Tyr Thr Leu Pro Pro Ser Arg Asp Glu Leu Thr Lys Asn Gln
   305 310 315 320
   Val Ser Leu Thr Cys Leu Val Lys Gly Phe Tyr Pro Ser Asp Ile Ala
   325 330 335
   Val Glu Trp Glu Ser Asn Gly Gln Pro Glu Asn Asn Tyr Lys Thr Thr
   340 345 350
   Pro Pro Val Leu Asp Ser Asp Gly Ser Phe Phe Leu Tyr Ser Lys Leu
   355 360 365
   Thr Val Asp Lys Ser Arg Trp Gln Gln Gly Asn Val Phe Ser Cys Ser
   370 375 380
   Val Met His Glu Ala Leu His Asn His Tyr Thr Gln Lys Ser Leu Ser
   385 390 395 400
   Leu Ser Pro Gly
<210> 8
   <211> 401
   <212> PRT
   <213> Human
<400> 8
   Met Asp Lys Thr His Thr Cys Pro Pro Cys Pro Ala Pro Glu Leu Leu
    1               5                  10                  15
   Gly Gly Pro Ser Val Phe Leu Phe Pro Pro Lys Pro Lys Asp Thr Leu
               20                  25                  30
   Met Ile Ser Arg Thr Pro Glu Val Thr Cys Val Val Val Asp Val Ser
           35                  40                  45
   His Glu Asp Pro Glu Val Lys Phe Asn Trp Tyr Val Asp Gly Val Glu
       50                  55                  60
   Val His Asn Ala Lys Thr Lys Pro Arg Glu Glu Gln Tyr Asn Ser Thr
   65                  70                  75                  80
   Tyr Arg Val Val Ser Val Leu Thr Val Leu His Gln Asp Trp Leu Asn
                   85                  90                  95
   Gly Lys Glu Tyr Lys Cys Lys Val Ser Asn Lys Ala Leu Pro Ala Pro
               100                 105                 110
   Ile Glu Lys Thr Ile Ser Lys Ala Lys Gly Gln Pro Arg Glu Pro Gln
           115                 120                 125
   Val Tyr Thr Leu Pro Pro Ser Arg Asp Glu Leu Thr Lys Asn Gln Val
   130 135 140
   Ser Leu Thr Cys Leu Val Lys Gly Phe Tyr Pro Ser Asp Ile Ala Val
   145 150 155 160
   Glu Trp Glu Ser Asn Gly Gln Pro Glu Asn Asn Tyr Lys Thr Thr Pro
   165 170 175
   Pro Val Leu Asp Ser Asp Gly Ser Phe Phe Leu Tyr Ser Lys Leu Thr
   180 185 190
   Val Asp Lys Ser Arg Trp Gln Gln Gly Asn Val Phe Ser Cys Ser Val
   195 200 205
   Met His Glu Ala Leu His Asn His Tyr Thr Gln Lys Ser Leu Ser Leu
   210 215 220
   Ser Pro Gly Lys Glu Thr Phe Pro Pro Lys Tyr Leu His Tyr Asp Glu
   225 230 235 240
   Glu Thr Ser His Gln Leu Leu Cys Asp Lys Cys Pro Pro Gly Thr Tyr
   245 250 255
   Leu Lys Gln His Cys Thr Ala Lys Trp Lys Thr Val Cys Ala Pro Cys
   260 265 270
   Pro Asp His Tyr Tyr Thr Asp Ser Trp His Thr Ser Asp Glu Cys Leu
   275 280 285
   Tyr Cys Ser Pro Val Cys Lys Glu Leu Gln Tyr Val Lys Gln Glu Cys
   290 295 300
   Asn Arg Thr His Asn Arg Val Cys Glu Cys Lys Glu Gly Arg Tyr Leu
   305 310 315 320
   Glu Ile Glu Phe Cys Leu Lys His Arg Ser Cys Pro Pro Gly Phe Gly
   325 330 335
   Val Val Gln Ala Gly Thr Pro Glu Arg Asn Thr Val Cys Lys Arg Cys
   340 345 350
   Pro Asp Gly Phe Phe Ser Asn Glu Thr Ser Ser Lys Ala Pro Cys Arg
   355 360 365
   Lys His Thr Asn Cys Ser Val Phe Gly Leu Leu Leu Thr Gln Lys Gly
   370 375 380
   Asn Ala Thr His Asp Asn Ile Cys Ser Gly Asn Ser Glu Ser Thr Gln
   385 390 395 400
   Lys

## Description

### Field of the Invention

The present invention relates to the use of OPG for the preparation of a medicament for the prevention and/or treatment of bone loss associated with cancer. The invention also relates to the use of OPG for preparing compositions for the treatment of multiple myeloma.

### Background of the Invention

Many cancers can become established in tissues and organs which are far removed from the original site of tumor growth. Such cancers, termed metastatic cancers, can cause widespread complications that are often fatal. The skeleton is a common site for the spread of solid tumors, exceeded in frequency by only the liver and the lung. As a result of invasion by cancer cells, osteoclasts, the primary cells in bone that promote bone resorption, become hyperactivated and begin to break down bone at an accelerated rate. Osteoclasts are activated by substances such as parathyroid hormone-related peptide (PTHrP) and interleukin-1 (IL-1), both of which are increased in the bone microenvironment and are also produced by tumor cells. Patients with bone cancer frequently develop lytic bone lesions as a result of increased osteoclast activity. This condition is referred to as osteolytic bone metastasis. Bone lysis can lead to pathologic fractures, spinal collapse, hypercalcemic events and bone pain and is a major cause of mortality and morbidity. Alternatively, as in prostate cancer bone metastases, increased osteoclastic bone destruction is accompanied by increased but disorganized bone formation (Kylmaelae et al. Brit. J. Cancer 71, 1061-1064 (1995)). The original bone is removed, and replaced by woven unstructured bone so that the architectural integrity of the bone is lost. This also results in bone pain and other morbidities.

In addition osteoclast activity may increase the propensity of cancer cells to metastasize to bone and then to grow in that environment. Osteoclasts have been shown to release cytokines such as IL-6 which is a growth factor for some hematologic tumor cells such as multiple myeloma cells (O"Keefe et al. Lab. Invest. 76, 457-465 (1997)). In addition, osteoclasts have been shown to release growth factors from bone matrix during bone resorption. These include fibroblast growth factors and transforming growth factor β which are known to promote growth of many solid tumors. In this way osteoclastic activity could create a fertile environment for metastatic seeding within bone, and as the tumor cells begin to grow and promote bone resorption, cause release of growth factors from the bone to sustain tumor expansion.

Currently available cancer therapy agents can reduce or inhibit tumor growth but have little effect on underlying lytic bone disease. It has been reported that some chemotherapeutic regimens actually contribute to bone loss associated with hematological malignancies such as multiple myeloma and Hodgkin's disease and in the case of gonadotrophin releasing hormone receptor agonists. In addition, once cancer has spread to the bone, it becomes more difficult to treat using current regimens. It is therefore desirable to be able to prevent the development of bone metastases and to treat bone metastases to prevent bone loss at an early stage.

Bone anti-resorptive agents inhibit the number and/or activity of osteoclasts and reduce the rate at which bone is broken down. Such agents may be useful in preventing and/or treating bone resorption associated with bone cancer. It has been reported that bisphosphonates such as risedronate, ibandronate and pamidronate, which are anti-resorptive compounds, can reduce the severity of skeletal events (e.g., pathological fractures, spinal collapse, radiation of or surgery on bone) in mouse tumor models and in patients suffering from breast cancer and multiple myeloma and other tumor bone metastases. In addition bisphosphonates have been reported to reduce bone pain and other skeletal events in prostate cancer bone metastases. However, bisphosphonates have been shown to have limited efficacy with only a modest reduction in skeletal events even when given in high doses by infusion. When taken orally, bisphosphonates have reduced efficacy and can cause gastrointestinal irritation (e.g., heartburn, dyspepsia and nausea) and in some cases esophageal ulcers if not administered properly.

Osteoprotegerin (OPG) has been described in PCT Publication No. WO97/23614 and found to negatively regulate formation of osteoclasts in vitro and in vivo. OPG dramatically increased the bone density in transgenic mice expressing the OPG polypeptide and reduced the extent of bone loss when administered to ovariectomized rats. An analysis of OPG activity in in vitro osteoclast formation revealed that OPG blocks the differentiation of osteoclasts from monocyte/macrophage precursors. OPG appears to have specificity in regulating the extent of osteoclast formation. OPG is a potent factor in blocking bone resorption and may be used in the prevention and treatment of loss of bone mass. In vitro and in vivo activity of inhibiting osteoclast formation and blocking loss of bone was also observed in fusion proteins comprising OPG and an Fc domain.

Consequently, it is an object of the invention to provide alternative compositions for the treatment of bone loss associated with cancer that overcome many of the problems associated with current therapy.

It is a further object of the invention to provide alternative compositions for the prevention of bone loss associated with cancer by prophylactic treatment to decrease the incidence of bone metastasis and/or to delay the onset of bone metastasis.

It is a further object of the invention to provide alternative compositions to prevent and/or treat multiple myeloma.

### Summary of the Invention

The invention provides for the use of an OPG polypeptide for the preparation of a medicament for the prevention or treatment of bone loss associated with cancer in a mammal, wherein the cancer is selected from the group consisting of breast cancer, prostate cancer, thyroid cancer, cancer of the kidney, lung cancer, esophageal cancer, rectal cancer, bladder cancer, cervical cancer, ovarian cancer, liver cancer, cancer of the gastrointestinal tract, multiple myeloma and colon adenocarcinoma.

Also included is metastatic bone disease that increases both bone resorption and bone formation resulting in osteosclerotic bone metastases or mixed lytic and osteosclerotic metastases which are associated with bone pain and the loss of the structural integrity of bone as in tumors such as prostate cancer.

The use according to the claims also provides for preventing metastasis of cancer to bone upon administration a therapeutically effective amount of an OPG polypeptide.

The use according to the claims also provides for preventing or treating a metastatic bone disease in a mammal by preparing a medicament comprising a therapeutically effective amount of an OPG polypeptide in combination with a cancer therapy agent. The cancer therapy agent may be any agent which is used to treat tumor growth including radiation therapy and chemotherapeutic drugs. Examples of such agents include anthracyclines, taxol, tamoxifen, antibodies, such as anti-Her2 or anti-CD20 antibodies, and receptor agonists and antagonists, such as luteinizing hormone-releasing hormone (LHRH) antagonists. OPG polypeptide compositions may be administered prior to, concurrent with, or subsequent to administration of a cancer therapy agent.

The invention also provides for the use of an OPG polypeptide for the preparation of a medicament for the prevention or treatment of multiple myeloma.

OPG polypeptides of the invention encompass those polypeptides which have the activity of inhibiting bone resorption and may be used to prevent and/or treat loss of bone mass or prevent osteosclerotic bone metastasis (replacement of structurally sound bone with disorganized structurally deficient bone). In preferred embodiments, OPG polypeptides are fusion proteins comprising OPG and a heterologus peptide or protein. Such fusion proteins can exhibit increased circulating half-lives and slower clearance times, thereby providing a more sustained anti-resorptive activity and less frequent administration. In one aspect, the heterologous protein is an immunoglobulin Fc region, or a variant, fragment or derivative thereof.

### Description of the Figures

Figure 1 shows the amino acid sequence of the hinge, CH2 and CH3 regions of human IgGγ1.
Figure 2 shows the amino acid sequence of human OPG [1-401].
Figure 3 shows the amino acid sequence of OPG[22-194]-FC.
Figure 4 shows the amino acid sequence of OPG[22-201]-Fc.
Figure 5 shows the amino acid sequence of OPG[22-1941-FcΔC.
Figure 6 shows the amino acid sequence of OPG[22-201]-FcΔC.
Figure 7 shows the amino acid sequence of OPG[22-194]-FcG₁₀.
Figure 8 shows and amino acid sequence of metFcΔC-OPG[22-194].

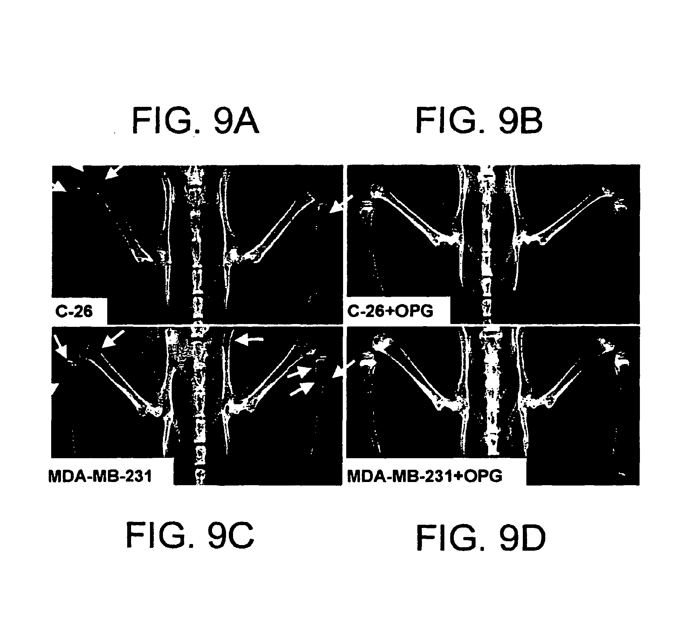

Figure 9 shows prevention of osteolytic bone destruction in C26-DCT and MDA-MB-231 models of tumor metastasis to bone. Both cells types produce localized bone destruction (yellow arrows) following inoculation directly into the left ventricle of mice. Panels on the left side show that radiographic lesions are evident 10 days after administration of C26-DCT cells and 28 days after administration of MDA-MB-231 cells. Panels on the right side show prevention of lesion formation after treatment with metFcΔC-OPG[22-194] (25mg/kg) every 3 days for 10 days (for C26-DCT mice) or 3 times/week for 4 weeks (for MDA-MB-231 mice).
Figure 10 shows a metFcΔC-OPG [22-194] dose dependent reduction in the number of radiographically evident osteolytic foci in mice inoculated with C26-DCT cells.
Figures 11A and 11B show prevention and reversal of hypercalcemia associated with malignancy in a mouse C26-DCT tumor model. In the prevention study, met FcΔC-OPG[22-194] was given by daily subcutaneous injection. In the reversal study, met FcΔC-OPG[22-194] was given by a single intravenous injection. Mean blood calcium levels ± SEM are reported.

### Detailed Description of the Invention

The present invention provides for compositions for the prevention and treatment of bone loss associated with cancer. The present invention also provides for the prevention and treatment of cancer using an anti-resorptive bone agent. Preferred compositions and uses of the invention include OPG and OPG fusion polypeptides. More particularly, the present invention relates to the use of OPG and OPG fusion protein for the preparation of a medicament for the prevention and/or treatment of cancer or for the prevention and/or treatment of bone loss associated with cancer.

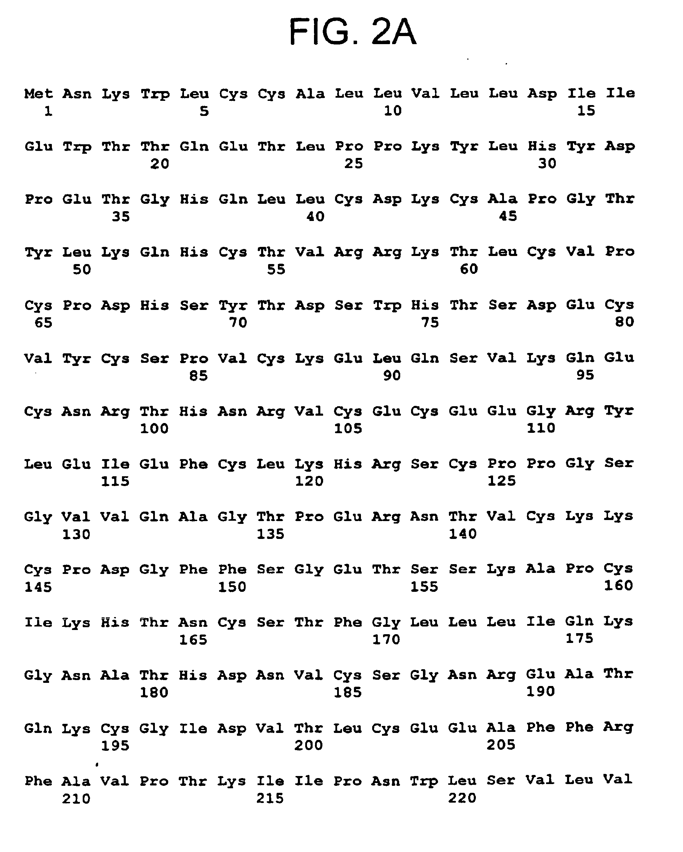

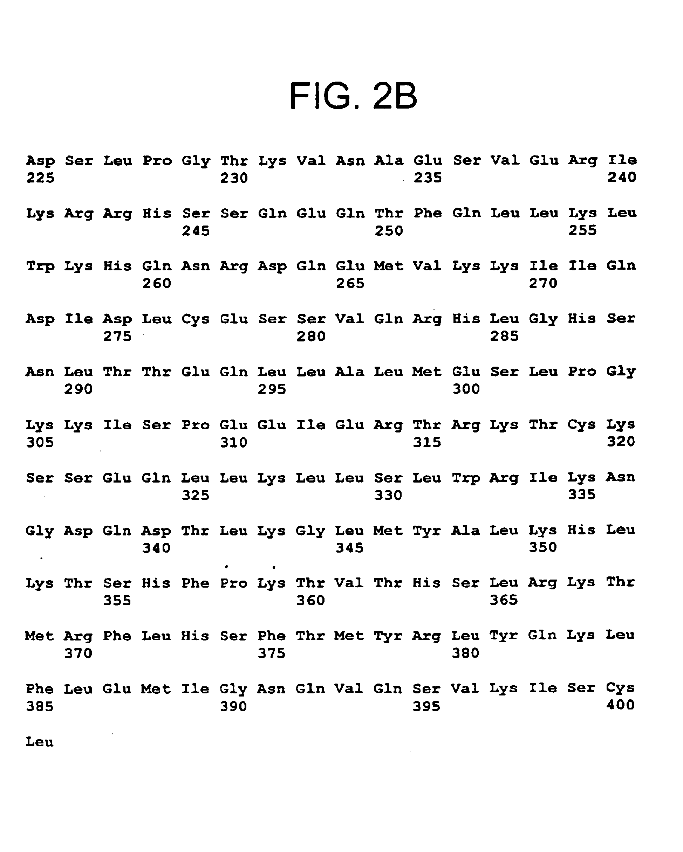

Unexpectedly, it has been observed that fusion of an Fc region to a truncated OPG polypeptide demonstrates advantages which are not seen with unfused truncated OPG polypeptides or with full-length mature OPG. (wherein full-length mature OPG has 380 amino acids, such as from residues 22 to 401 inclusive, as shown in Figure 2 (SEQ ID NO: 2) It has been further observed that fusion of an Fc region at the carboxy terminus of an OPG polypeptide provides unexpected advantages compared to fusion of an Fc region at the amino terminus of an OPG polypeptide. Accordingly, OPG fusion proteins, and variants, fragments and derivatives thereof, as well as, related methods of use and preparation, are described in more detail below.

The term "OPG" or "OPG polypeptide" refers to a polypeptide comprising the amino acid sequence as set forth in Figure 2 (SEQ ID NO: 2) and related polypeptides described herein. Related polypeptides include allelic variants; splice variants; fragments; derivatives; substitution, deletion and insertion variants; fusion polypeptides; and non-human homologs. OPG polypeptides may be mature polypeptides, as defined herein, which may or may not have an amino terminal methionine residue, depending upon the method of preparation.

The term "OPG fusion protein" refers to an OPG protein, or OPG polypeptide which is joined to a heterologous peptide or polypeptide. The OPG fusion proteins of the invention may be prepared by any suitable means known in the art, such as by genetic or chemical fusion of OPG and heterologous peptide or polypeptide moieties. In an embodiment of the invention, the heterologous peptide or polypeptide is an Fc region of an immunoglobulin, preferably a human immunoglobulin. A heterologous peptide or protein may be joined either to the amino terminus or to the carboxy terminus of an OPG polypeptide.

The term "mature OPG polypeptide" or "mature OPG fusion polypeptide" refers to a polypeptide or a fusion polypeptide lacking a leader sequence and may also include other modifications such as proteolytic processing of the amino terminus (with or without a leader sequence) and/or the carboxy terminus, cleavage of a smaller polypeptide from a larger precursor, N-linked and/or O-linked glycosylation, and the like.

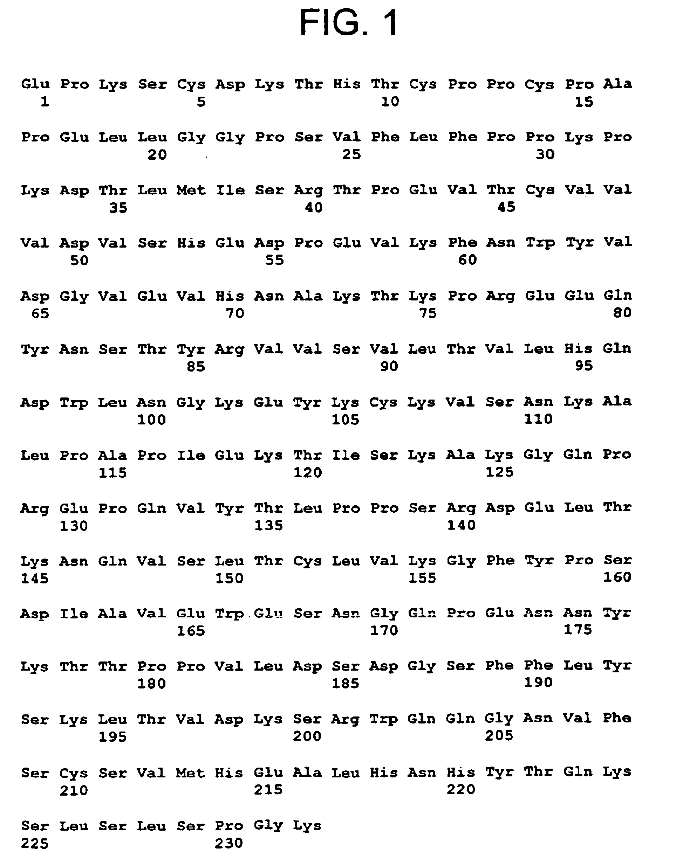

The term "Fc" refers to a molecule or sequence comprising the sequence of a non-antigen-binding portion of antibody, whether in monomeric or multimeric form. The original immunoglobulin source of an Fc is preferably of human origin and may be from any isotype, e.g., IgG, IgA, IgM, IgE or IgD. One method of preparation of an isolated Fc molecule involves digestion of an antibody with papain to separate antigen and non-antigen binding portions of the antibody. Another method of preparation of an isolated Fc molecules is production by recombinant DNA expression followed by purification of the Fc molecules so expressed. A full-length Fc consists of the following Ig heavy chain regions: C_{H}1, C_{H}2 and C_{H}3 wherein the C_{H}1 and C_{H}2 regions are typically connected by a flexible hinge region. In one embodiment, an Fc has the amino acid sequence of IgG₁ such as that shown in Figure 1. The terms "Fc protein, "Fc sequence", "Fc molecules, "Fc region" and "Fc portion" are taken to have the same meaning as "Fc".

The term "fragment" when used in association with Fc or OPG polypeptides, or with fusion polypeptides thereof, refers to a peptide or polypeptide that comprises less than the full length amino acid sequence of an Fc or OPG polypeptide. Such a fragment may arise, for example, from a truncation at the amino terminus, a truncation at the carboxy terminus, and/or an internal deletion of a residue(s) from the amino acid sequence. OPG or Fc fragments may result from alternative RNA splicing or from *in vivo* protease activity.

The term "variant" when used in association with Fc or OPG polypeptides, or with fusion polypeptides thereof, refers to a polypeptide comprising an amino acid sequence which contain one or more amino acid sequence substitutions, deletions, and/or additions as compared to native Fc or OPG polypeptide amino acid sequences. Variants may be naturally occurring or artificially constructed. Variants of the invention may be prepared from the corresponding nucleic acid molecules encoding said variants, which have a DNA sequence that varies accordingly from the DNA sequences for native Fc or OPG polypeptides.

The term "derivative" when used in association with Fc or OPG polypeptides, or with fusion polypeptides thereof, refers to Fc or OPG polypeptide variants or fragments thereof, that have been chemically modified, as for example, by covalent attachment of one or more polymers, including, but limited to, water soluble polymers, N-linked or O-linked carbohydrates, sugars, phosphates, and/or other such molecules. The derivatives are modified in a manner that is different from native Fc or OPG, either in the type or location of the molecules attached to the polypeptide. Derivatives further includes deletion of one or more chemical groups naturally attached to an Fc or OPG polypeptide.

The term "fusion" refers to joining of different peptide or protein segments wherein the joined ends of the peptide or protein segments may be directly adjacent to each other or may be separated by linker or spacer moieties such as amino acid residues or other linking groups. A fusion may be accomplished by genetic or chemical means using procedures available to one skilled in the art although the means of joining is not limited to those disclosed herein.

### Polypeptides

The use according to the invention provides for OPG fusion polypeptides and compositions thereof, and more particularly provides for fusion polypeptides comprising OPG and Fc moieties. Fusions of an Fc region to an OPG polypeptide may be made at the amino terminus of OPG, that is, the carboxy terminus of an Fc region is fused to the amino terminus of OPG. These fusion proteins (and nucleic acids encoding same) are designated herein as FcOPG. It may also be desirable to fuse the carboxy terminus of OPG to the amino terminus of an Fc region. These fusion proteins (and nucleic acids encoding same) are designated herein as OPGFC.

An Fc, or a variant, fragment or derivative thereof, may be from an immunoglobulin (Ig) class. In one embodiment, an Fc is from the IgG class, such as IgG₁, IgG₂, IgG₃, and IgG₄. In another embodiment, an Fc is from IgG₁. An Fc may also comprise amino acid residues represented by a combination of any two or more of the Ig classes, such as residues from IgG₁ and IgG₂, or from IgG₁, IgG₂ and IgG₃, and so forth. In one embodiment, an Fc region of an OPG fusion protein has the sequence as set forth in Figure 1 (SEQ ID NO:1) comprising hinge C_{H}2 and C_{H}3 regions of human IgG1. (see Ellison *et al.,* Nucleic Acids Res. 10, 4071-4079 (1982).

In addition to naturally occurring variations in Fc regions, Fc variants, fragments and derivatives may contain non-naturally occurring changes in Fc which are constructed by, for example, introducing substitutions, additions, insertions or deletions of residues or sequences in a native or naturally occurring Fc, or by modifying the Fc portion by chemical modification and the like. In general, Fc variants, fragments and derivatives are prepared such that the increased circulating half-life of Fc fusions to OPG is largely retained.

Also provided by the use according to the invention are Fc and OPG variants with conservative amino acid substitutions. The term "conservative amino acid substitution" refers to a substitution of a native amino acid residue with a nonnative residue such that there is little or no effect on the polarity or charge of the amino acid residue at that position. For example, a conservative substitution results from the replacement of a non-polar residue in a polypeptide with any other non-polar residue. General rules for conservative amino acid substitutions are set forth in Table I.

**Table I**

| Conservative Amino Acid Substitutions | | |
|---|---|---|
| Original Residues | Exemplary Substitutions | Preferred Substitutions |
| Ala | Val,Leu,Ile | Val |
| Arg | Lys,Gln,Asn | Lys |
| Asn | Gln,His,Lys,Arg | Gln |
| Asp | Glu | Glu |
| Cys | Ser | Ser |
| Gln | Asn | Asn |
| Glu | Asp | Asp |
| Gly | Pro,Ala | Ala |
| His | Asn,Gln,Lys,Arg | Arg |
| Ile | Leu,Val,Met,Ala,Phe,Norleucine | Leu |
| Leu | Norleucine,Ile,Val,Met,Ala,Phe | Ile |
| Lys | Arg,Gln,Asn | Arg |
| Met | Leu,Phe,Ile | Leu |
| Phe | Leu,Val,Ile,Ala,Tyr | Leu |
| Pro | Ala | Ala |
| Ser | Thr | Thr |
| Thr | Ser | Ser |
| Trp | Tyr,Phe | Tyr |
| Tyr | Trp,Phe,Thr,Ser | Phe |
| Val | Ile,Met,Leu,Phe,Ala,Norleucine | Leu |

Conservative amino acid substitutions also encompass non-naturally occurring amino acid residues which are typically incorporated by chemical peptide synthesis rather than by synthesis in biological systems. These include peptidomimetics, and other reversed or inverted forms of amino acid moieties. Conservative modifications to the amino acid sequence (and the corresponding modifications to the encoding nucleotides) are expected to produce Fc and OPG molecules (and OPG fusion proteins) having functional and chemical characteristics similar to those of unmodified Fc, OPG and OPG fusion proteins.

In addition to the substitutions set forth in Table I, any native residue in an Fc region or in an OPG polypeptide (or in an FcOPG fusion protein) may also be substituted with alanine, as has been previously described for "alanine scanning mutagenesis" (Cunningham et al. Science 244, 1081-1085 (1989)).

Substantial modifications in the functional and/or chemical characteristics of an Fc or OPG polypeptide (and an OPG fusion protein) may be accomplished by selecting substitutions that differ significantly in their effect on maintaining (a) the structure of the molecular backbone in the area of the substitution, for example, as a sheet or helical conformation, (b) the charge or hydrophobicity of the molecule at the target site, or (c) the bulk of the side chain. Naturally occurring residues may be divided into groups based on common side chain properties:
1) hydrophobic: norleucine, Met, Ala, Val, Leu, Ile;
2) neutral hydrophilic: Cys, Ser, Thr;
3) acidic: Asp, Glu;
4) basic: Asn, Gln, His, Lys, Arg;
5) residues that influence chain orientation: Gly, Pro; and
6) aromatic: Trp, Tyr, Phe.

Non-conservative substitutions may involve the exchange of a member of one of these classes for a member from another class. Such substituted residues may be introduced into regions of an Fc or OPG molecule that are homologous with non-human Fc or OPG, or into the non-homologous regions of the molecule.

Cysteine residues in Fc molecules can be deleted or replaced with other amino acids to prevent formation of disulfide crosslinks. In particular, a cysteine residue at position 5 of Figure 1 (SEQ. ID. NO. 1) may be substituted with one or more amino acids, such as alanine or serine. Alternatively, the cysteine residue at position 5 could be deleted.

An Fc fragment may be prepared by deletion of one or more amino acids at any of positions 1, 2, 3, 4 and 5 as shown in Figure 1 (SEQ ID NO. 1). In one Fc molecule, FcΔC, the amino acid residues at positions 1-5 inclusive are deleted. Substitutions at these positions can also be made and are with in the scope of this invention.

Fc variants may also be made which show reduced binding to Fc receptors which trigger effector functions such as antibody dependent cellular cytotoxicity (ADCC) and activation of complement. Such variants may include leucine at position 20 deleted or substituted with a glutamine residue, glutamate at position 103 deleted or substituted with an alanine residue, and lysines at positions 105 and 107 deleted or substituted with alanine residues (following the numbering as set forth in Figure 1). One or more of such substitutions are contemplated.

In one embodiment, Fc variants will exhibit stronger binding to the FcRn receptor ("salvage receptor") and a longer circulating half-life compared to native Fc. Examples of such variants include amino acid substitutions at one or more of residues 33, 35-42, 59, 72, 75, 77, 95-98, 101, 172-174, 215 and 220-223 as shown in Figure 1 (SEQ ID NO: 1), wherein the substitution(s) confer tighter binding of an Fc variant to the FcRn receptor.

Other Fc variants include one or more tyrosine residues replaced with, for example, phenylalanine residues. In addition, other variant amino acid insertions, deletions and/or substitutions are also contemplated and are within the scope of the use of the present invention. Examples include Fc variants disclosed in WO96/32478 and WO97/34630. Furthermore, alterations may be in the form of altered amino acids, such as peptidomimetics or D-amino acids.

An Fc protein may be also linked to an OPG moiety of the OPG fusion polypeptide by spacer or linker moieties. Such spacers or linkers may be proteinaceous in that they comprise one or more amino acids or they may be chemical linkers. Such chemical linkers are well known in the art. Amino acid linker sequences can include but are not limited to:
(a) ala-ala-ala;
(b) ala-ala-ala-ala;
(c) ala-ala-ala-ala-ala;
(d) gly-gly;
(e) gly-gly-gly;
(f) gly-gly-gly-gly-gly;
(g) gly-gly-gly-gly-gly-gly-gly;
(h) gly-pro-gly;
(i) gly-gly-pro-gly-gly;
(j) val;
(k) ser-gly-gly-gly-gly-gly-gly-gly-gly;
(l) gly-gly-ser-gly-ser-gly-ala-gly-ser-gly-ser-gly-gly-gly-ser-gly-ser-gly-gly;
(m) a chemical moiety; and
(n) any combination of subparts (a) through (m).

OPG variants, fragments and derivatives are also provided by the invention and are generally as described hereinabove for Fc molecules, with the exception of the specific locations of the modified amino acid residues. OPG variants, fragments and derivatives are described in PCT WO97/23614.

In a preferred embodiment, the OPG moiety of an OPG fusion protein is a carboxy-terminal truncated form of OPG. Carboxy terminal truncated forms of OPG have one or more amino acids from positions 186-401 as shown in Figure 2 deleted. For example, OPG truncations comprise the amino acid sequence 22-X wherein X is any residue from 185 to 400 inclusive. In another embodiment, OPG truncations comprise the amino acid sequence 22-X wherein X is any residue from 185 to 278 inclusive, or from 185 to 293 inclusive , or alternatively, from 194 to 278 inclusive, or from 194 to 293 inclusive. Fusion proteins comprising the OPG truncated polypeptides described herein encompass joining of the OPG and heterologous peptide or polypeptide moieties directly or through a spacer or linker molecule wherein the spacer or linker optionally comprises one or more amino acid residues. Variants and derivatives of the OPG truncated forms described herein are also encompassed by the invention.

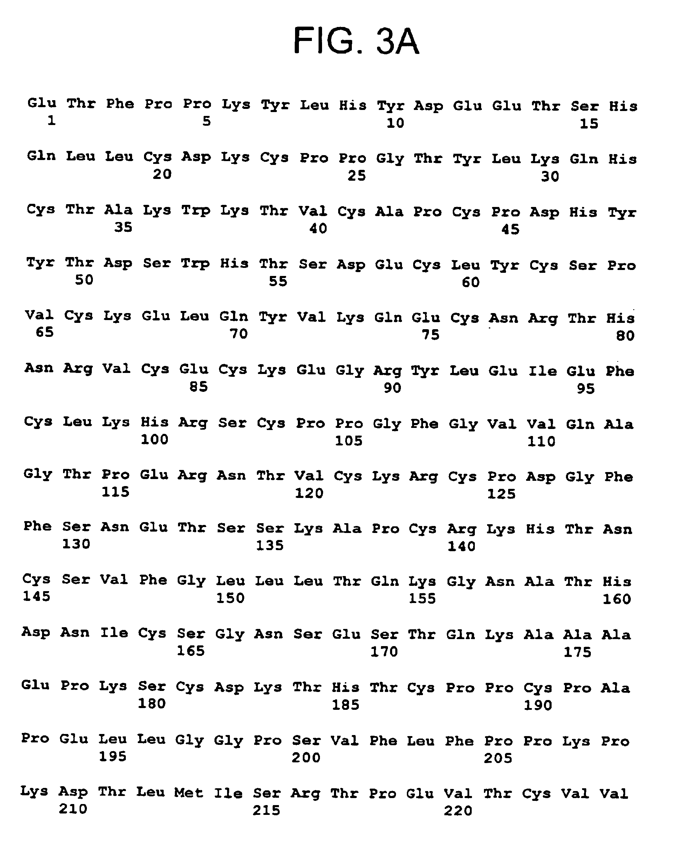

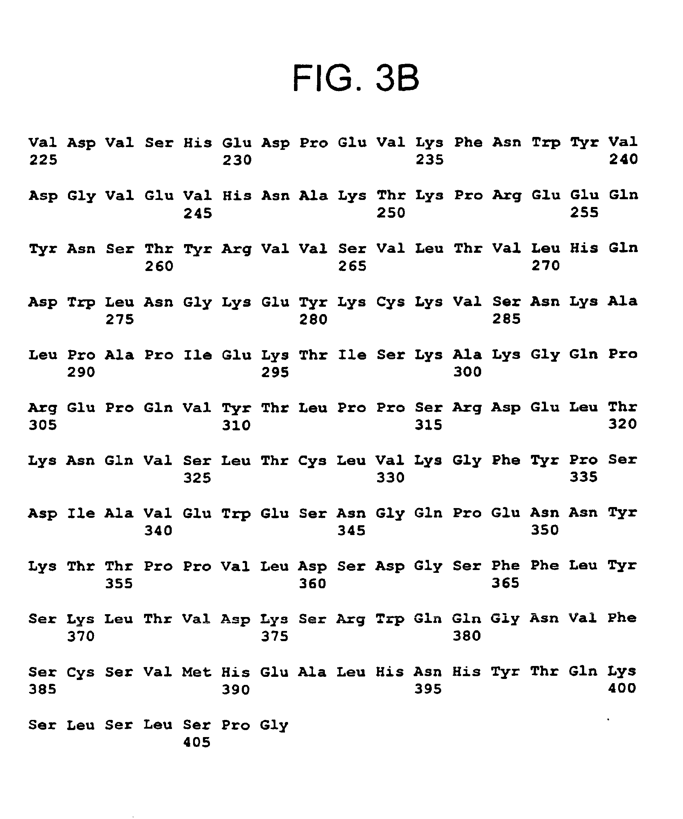

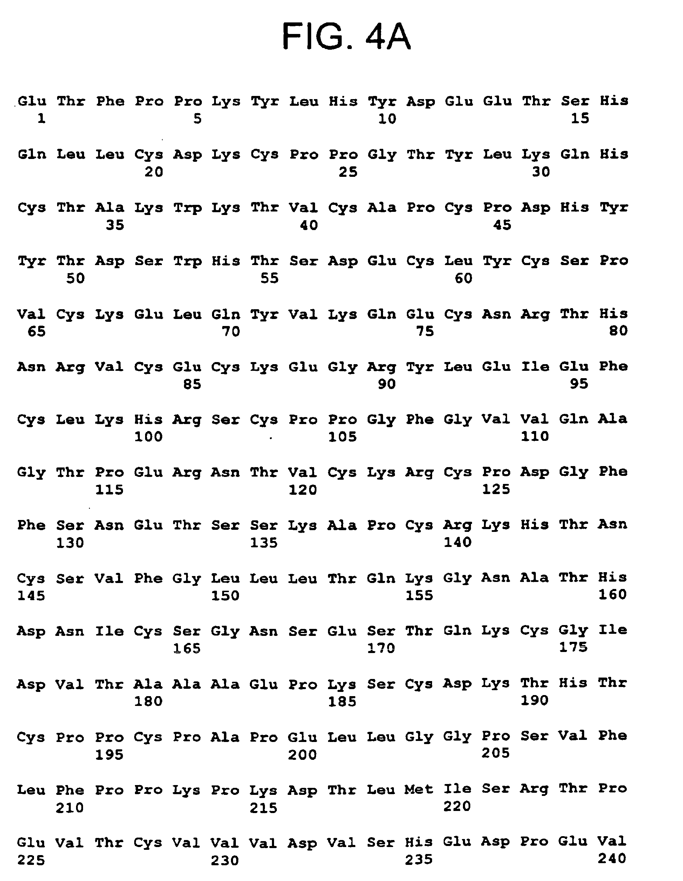

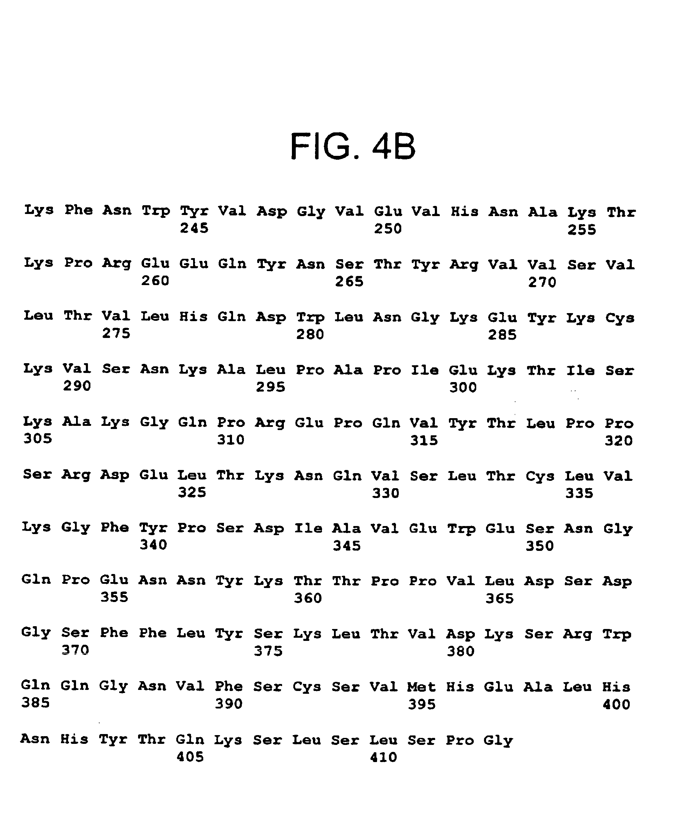

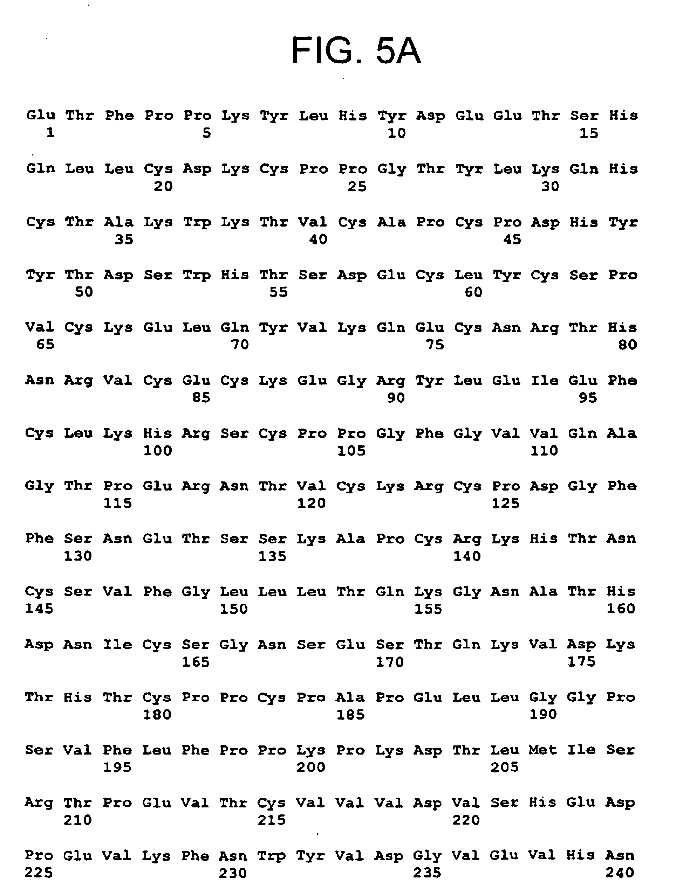

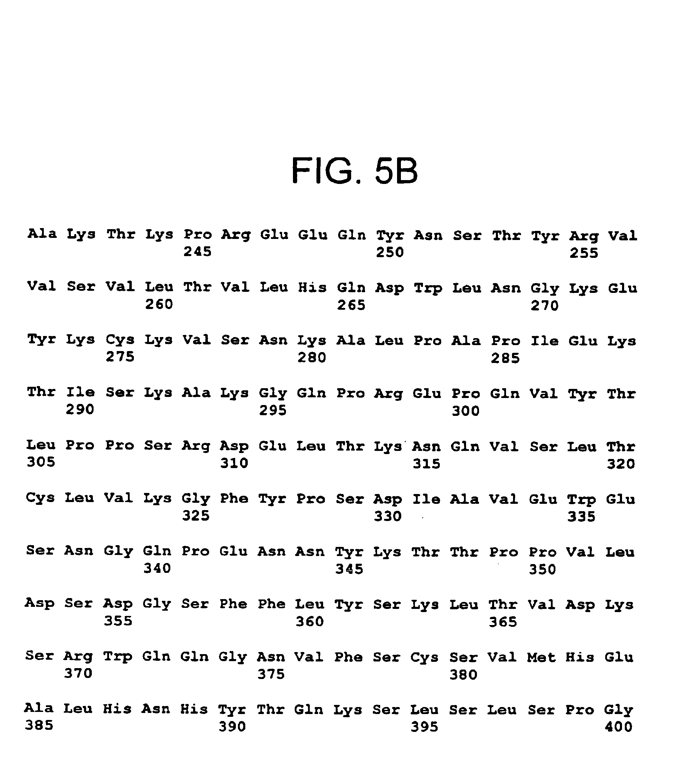

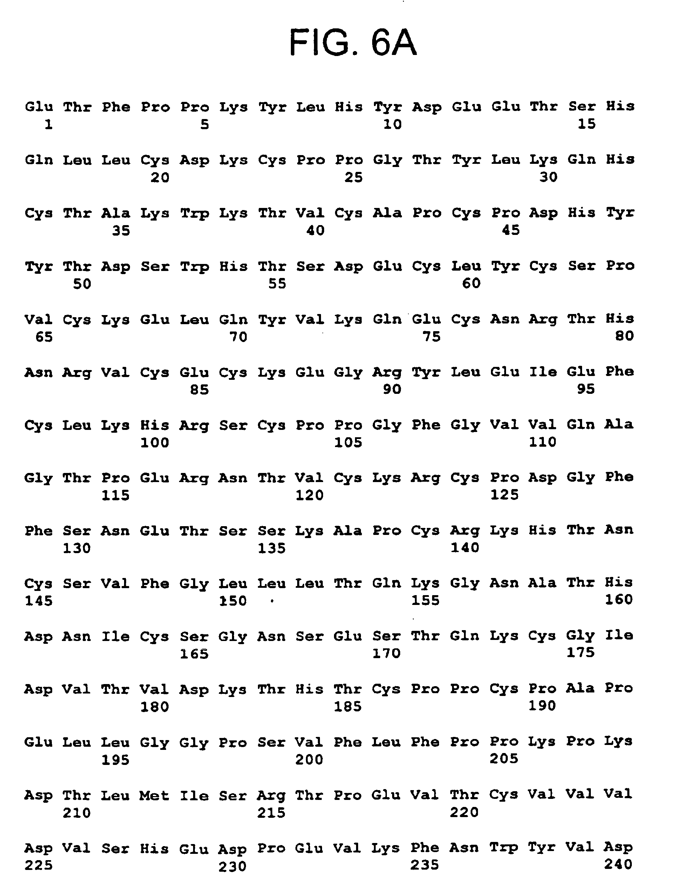

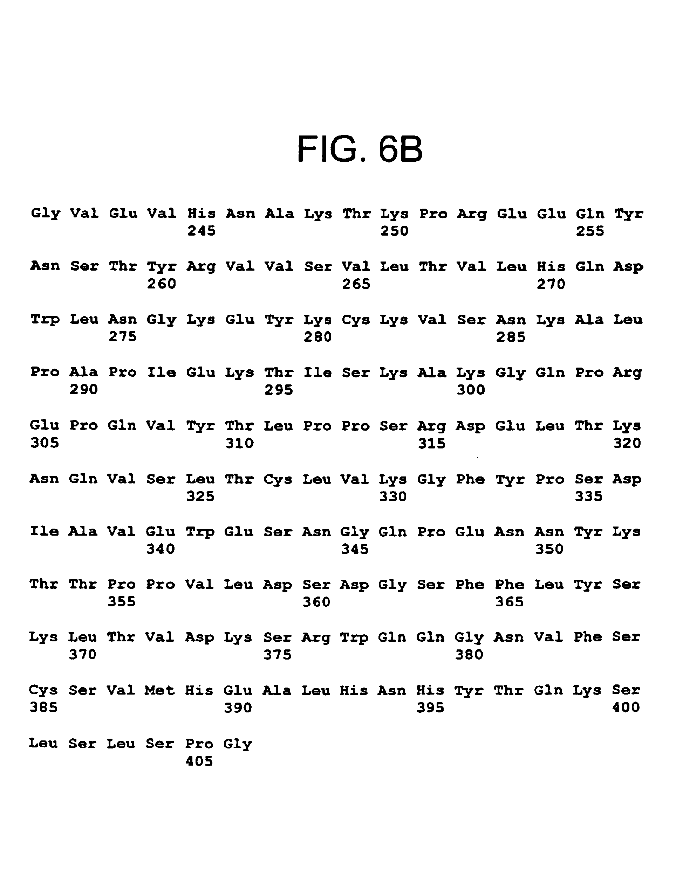

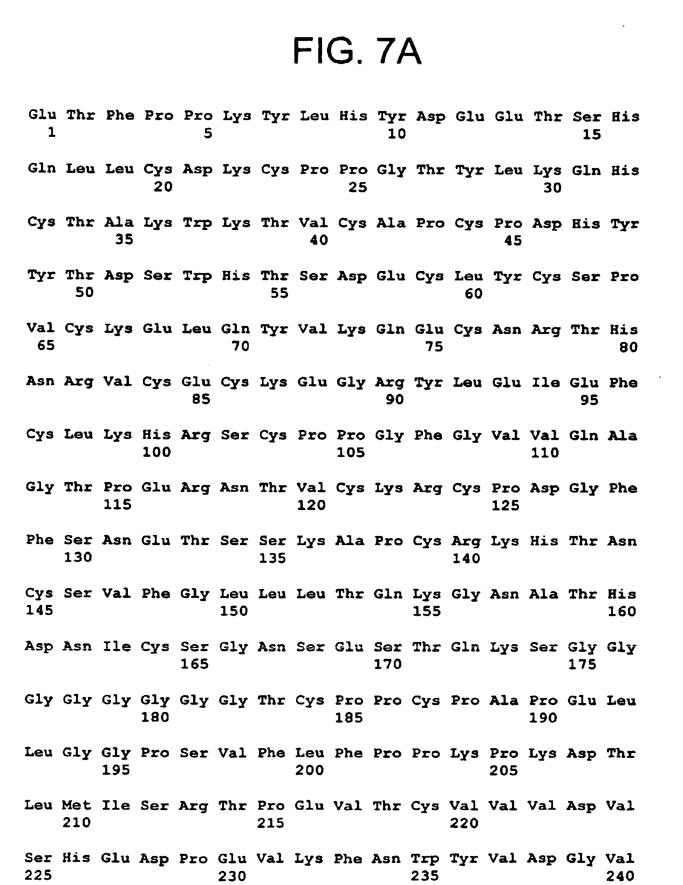

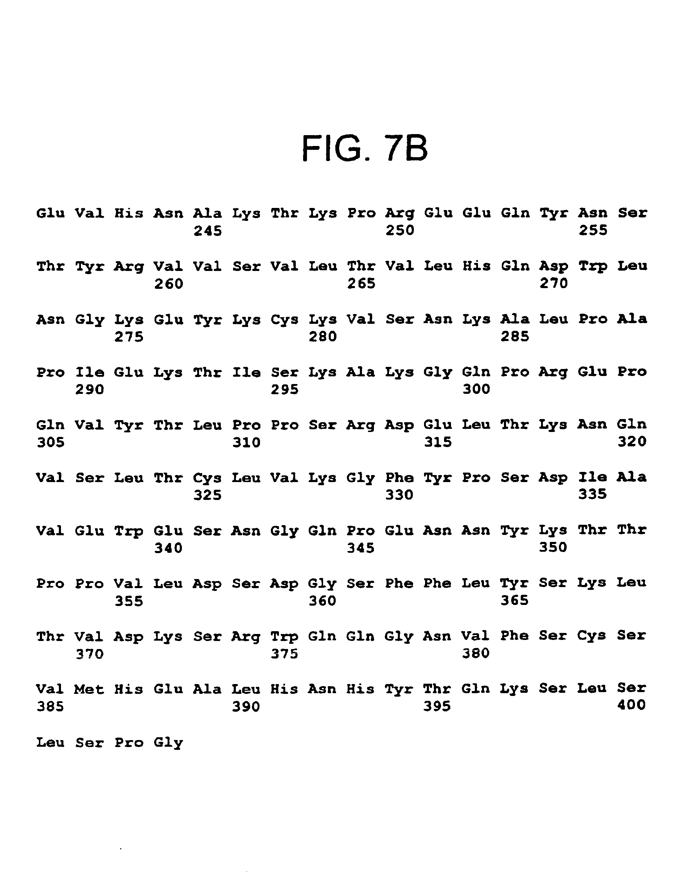

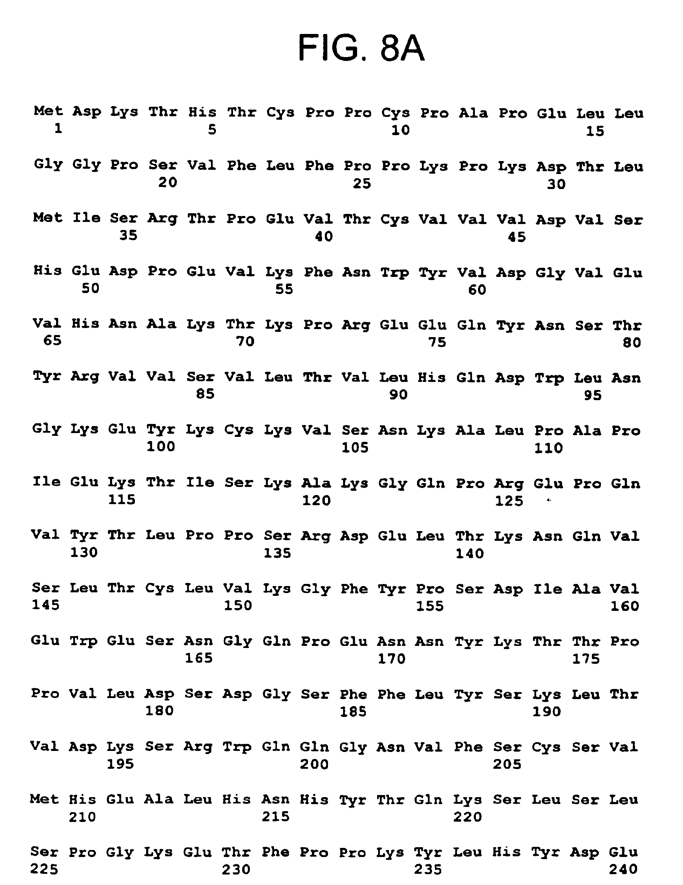

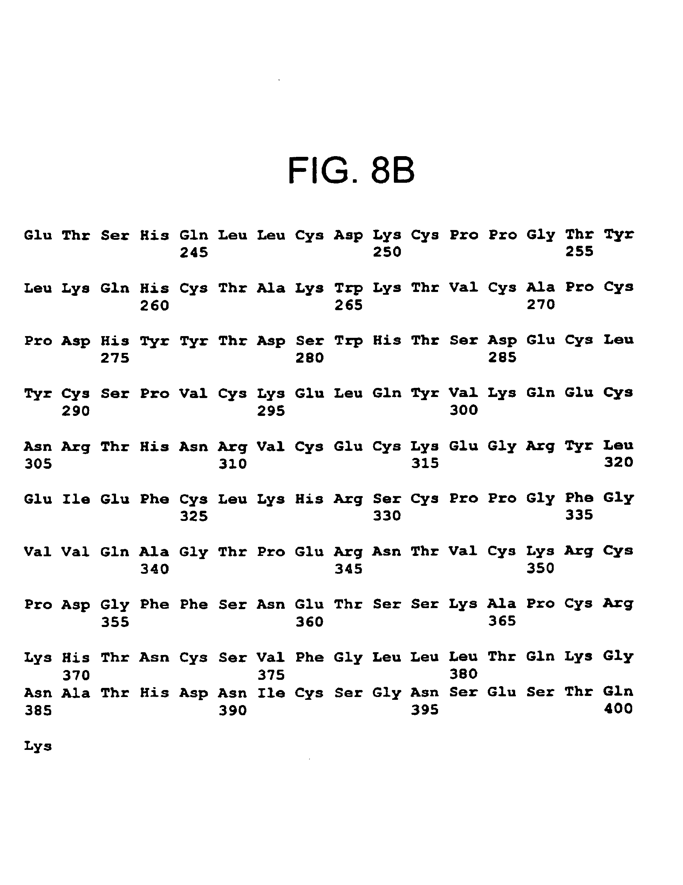

Preferred fusion proteins of the use of the present invention include those wherein the OPG moiety comprises the amino acid sequence 22-X wherein X is any residue from positions 194 to 201 inclusive using the numbering as shown in Figure 2 (SEQ ID NO: 2). Examples of such fusion proteins include the following:
OPG [22-194]-Fc (Figure 3 and SEQ ID NO: 3)
OPG [22-201]-Fc (Figure 4 and SEQ ID NO: 4)
OPG [22-194]-FcΔC (Figure 5 and SEQ ID NO: 5)
OPG [22-201]-FcΔC (Figure 6 and SEQ ID NO: 6)
OPG [22-194]-FcG₁₀ (Figure 7 and SEQ ID NO: 7)
FcΔC-OPG [22-194] (Figure 8 and SEQ ID NO: 8)

For the preferred polypeptides, the term "Fc" refers to the sequence of human IgG₁ shown in Figure 1, the term "fcΔC" refers to the sequence shown in Figure 1 (SEQ ID NO: 1) lacking amino acid residues 1-5 inclusive, and the term "FcG₁₀" refers to an Fc moiety lacking amino acid residues 1-9 inclusive, and having a ser-(gly)₈ linker.

### Nucleic acid molecules

Nucleic acid molecules encoding OPG fusion proteins of the invention, or variants, fragments or derivatives thereof, are provided for by the invention. Nucleic acid molecules of the invention may be produced by recombinant DNA methodology known to one skilled in the art. See, for example, Sambrook et al. (Molecular Cloning: A Laboratory Manual, Cold Springs Harbor Laboratory Press, Cold Springs Harbor, N.Y. (1989)), and Ausubel et al. (Current Protocols in Molecular Biology, Wiley and Sons, N.Y. (1994)), for descriptions of mutagenesis techniques. Chemical synthesis using methods described by Engels et al. (Angew. Chem. Intl. Ed. 28, 716-734 (1989)), may also be used to prepare such variants. Other methods for preparing nucleic acid molecules known to the skilled artisan may also be used.

In certain embodiments, nucleic acid molecules encode OPG fusion protein variants with conservative amino acid substitutions as defined hereinabove. For example, conservative amino acid substitutions are made in an OPG and/or in an Fc moiety of a fusion protein. Also provided for are Fc or OPG variants comprising an addition and/or a deletion of one or more N-linked or O-linked glycosylation sites, or comprising Fc or OPG polypeptide fragments as described above. It is understood that the nucleic acid molecules of the invention may encode any combination of Fc and/or OPG variants, fragments, and fusion polypeptides described herein.

In another embodiment, nucleic acids of the invention contain codons which have been altered for optimal expression of an OPG fusion polypeptide in a given host cell. Particular codon alterations will depend upon the OPG fusion polypeptide(s) and host cell(s) selected for expression. Such "codon optimization" can be carried out by a variety of methods, for example, by selecting codons which are preferred for use in highly expressed genes in a given host cell. Computer algorithms which incorporate codon frequency tables such as "Ecohigh. Cod" for codon preference of highly expressed bacterial genes may be used and are provided by the University of Wisconsin Package version 9.0, Genetics Computer Group, Madison, WI. Other useful codon frequency tables include
"Celegans_high.cod", "Celegans_low.cod",
"Drosophila_high.cod", "Human_high.cod",
"Maize_high.cod", and "Yeast_high.cod".

### Vectors and Host cells

A nucleic acid molecule encoding an OPG fusion polypeptide is inserted into an appropriate expression vector using standard ligation techniques. The vector is typically selected to be functional in the particular host cell employed (*i.e.*, the vector is compatible with the host cell machinery such that amplification of the gene and/or expression of the gene can occur). A nucleic acid molecule encoding an OPG protein may be amplified/expressed in prokaryotic, yeast, insect (baculovirus systems) and/or eukaryotic host cells. Selection of the host cell will depend in part on whether an OPG protein is to be post-translationally modified (e.g, glycosylated and/or phosphorylated). If so, yeast, insect, or mammalian host cells are preferable.

Typically, expression vectors used in any of the host cells will contain sequences for plasmid maintenance and for cloning and expression of exogenous nucleotide sequences. such sequences, collectively referred to as "flanking sequences" in certain embodiments will typically include one or more of the following nucleotides: a promoter, one or more enhancer sequences, an origin of replication, a transcriptional termination sequence, a complete intron sequence containing a donor and acceptor splice site, a leader sequence for secretion, a ribosome binding site, a polyadenylation sequence, a polylinker region for inserting the nucleic acid encoding the polypeptide to be expressed, and a selectable marker element.

Flanking sequences may be homologous (i.e., from the same species and/or strain as the host cell), heterologous (i.e, from a species other than the host cell species or strain), hybrid (i.e., a combination of flanking sequences from more than one source), or synthetic, or native sequences which normally function to regulate OPG and/or Fc protein expression. As such, the source of flanking sequences may be any prokaryotic or eukaryotic organism, any vertebrate or invertebrate organism, or any plant, provided that the flanking sequences is functional in, and can be activated by, the host cell machinery.

A leader, or signal, sequence may be used to direct an OPG fusion polypeptide out of the host cell. The signal sequence is most commonly positioned directly at the 5' end of an OPG fusion polypeptide coding region. Many signal sequences have been identified, and any of them that are functional in the selected host cell may be used in conjunction with nucleic acid sequences encoding OPG fusion proteins. For example, a signal sequence may be homologous (naturally occurring) or heterologous to the OPG or Fc gene or cDNA, Additionally, a signal sequence may be chemically synthesized using methods set forth above. In most cases, secretion of an OPG polypeptide from the host cell via the presence of a signal peptide will result in the removal of the signal peptide from the fusion polypeptide.

The signal sequence may be a component of the vector, or it may be a part of OPG DNA that is inserted into the vector. For example, OPG DNA encodes a signal sequence at the amino terminus of the protein that is cleaved during post-translational processing of the molecule to form the mature protein (see Figure 2). Included within the scope of this invention are OPG nucleotides with the native signal sequence as well as OPG nucleotides wherein the native signal sequence is deleted and replaced with a heterologous signal sequence. A heterologous signal sequence selected should be one that is recognized and processed, i.e., cleaved by a signal peptidase, by the host cell. In one embodiment, a heterologous signal sequence is the OPG signal sequence as described in WO97/23614. For prokaryotic host cells that do not recognize and process the native OPG signal sequence, the signal sequence is substituted by a prokaryotic signal sequence selected, for example, from the group of the alkaline phosphatase, penicillinase, or heat-stable enterotoxin II leaders. For yeast secretion, the native OPG signal sequence may be substituted by the yeast invertase, alpha factor, or acid phosphatase leaders. In mammalian cell expression the native signal sequence is satisfactory, although other mammalian signal sequences may be suitable.

Preferred vectors for practicing this invention are those which are compatible with bacterial, insect, and mammalian host cells. Such vectors include, *inter alia,* pCRII, pCR3, and pcDNA3.1 (Invitrogen Company, San Diego, CA), pBSII (Stratagene Company, La Jolla, CA), pET15b (Novagen, Madison, WI), pGEX (Pharmacia Biotech, Piscataway, NJ), pEGFP-N2 (Clontech, Palo Alto, CA), pETL (BlueBacII; Invitrogen), pDSRa2 (PCT Publication No. WO90/14363) and pFastBacDual (Gibco/BRL, Grand Island, NY).

Additional possible vectors include, but are not limited to, cosmids, plasmids or modified viruses, but the vector system must be compatible with the selected host cell. Such vectors include, but are not limited to plasmids such as Bluescript plasmid derivatives (a high copy number ColE1-based phagemid, Stratagene Cloning Systems Inc., La Jolla CA), PCR cloning plasmids designed for cloning Taq-amplified PCR products (e.g., TOPO^{™} TA Cloning^{®} Kit, PCR2.1^{®} plasmid derivatives, Invitrogen, Carlsbad, CA), and mammalian , yeast or virus vectors such as a baculovirus expression system (pBacPAK plasmid derivatives, Clontech, Palo Alto, CA). The recombinant molecules can be introduced into host cells via transformation, transfection, infection, electroporation, or other known techniques. After the vector has been constructed and a nucleic acid molecule encoding an OPG fusion polypeptide has been inserted into the proper site of the vector, the completed vector may be inserted into a suitable host cell for amplification and/or polypeptide expression.

Host cells may be prokaryotic host cells (such as *E. coli*) or eukaryotic host cells (such as a yeast cell, an insect cell, or a vertebrate cell). The host cell, when cultured under appropriate conditions, synthesizes an OPG polypeptide which can subsequently be collected from the culture medium (if the host cell secretes it into the medium) or directly from the host cell producing it (if it is not secreted). Selection of an appropriate host cell will depend upon various factors, such as desired expression levels, polypeptide modifications that are desirable or necessary for activity, such as glycosylation or phosphorylation, and ease of folding into a biologically active molecule.

Suitable host cells or cell lines may be mammalian cells, such as Chinese hamster ovary cells (CHO) (ATCC #CCL61 and Urlaub et al., Proc. Natl. Acad. Sci. USA 77, 4216-4220 (1980)), human embryonic kidney (HEK) 293 or 293T cells (ATCC #CRL1573), or 3T3 cells (ATCC #CRL1658). The selection of suitable mammalian host cells and methods for transformation, culture, amplification, screening and product production and purification are known in the art. Other suitable mammalian cell lines, are the monkey COS-1 and COS-7 cell lines (ATCC #CRL1651), and the CV-1 cell line (ATCC #CCL70). Further exemplary mammalian host cells include primate cell lines and rodent cell lines, including transformed cell lines. Normal diploid cells, cell strains derived from *in vitro* culture of primary tissue, as well as primary explants, are also suitable. Candidate cells may be genotypically deficient in the selection gene, or may contain a dominantly acting selection gene. Other suitable mammalian cell lines include but are not limited to, mouse neuroblastoma N2A cells, HeLa, mouse L-929 cells, 3T3 lines derived from Swiss, Balb-c or NIH mice, BHK or HaK hamster cell lines. Each of these cell lines is known by and available to those skilled in the art.

Similarly useful as host cells suitable for the present invention are bacterial cells. For example, the various strains of *E. coli* (e.g., HB101, DH5a, DH10, and MC1061) are well-known as host cells in the field of biotechnology. Various strains of B. *subtilis, Pseudomonas spp.,* other *Bacillus spp., Streptomyces spp.,* and the like may also be employed in this method.

Many strains of yeast cells known to those skilled in the art are also available as host cells for expression of the polypeptides of the present invention. Preferred yeast cells include, for example, *Saccharomyces cerivisae.*

Additionally, where desired, insect cell systems may be utilized in the methods of the present invention. Such systems are described for example in Kitts et al. (Biotechniques, 14, 810-817 (1993)), Lucklow (Curr. Opin. Biotechnol., 4, 564-572 (1993))and Lucklow et al. (J. Virol., 67, 4566-4579 (1993)). Preferred insect cells are Sf-9 and Hi5 (Invitrogen, Carlsbad, CA).

Transformation or transfection of an expression vector for an OPG polypeptide into a selected host cell may be accomplished by well known methods including methods such as calcium chloride, electroporation, microinjection, lipofection or the DEAE-dextran method. The method selected will in part be a function of the type of host cell to be used. These methods and other suitable methods are well known to the skilled artisan, and are set forth, for example, in Sambrook et al., supra.

### Polypeptide Production

Host cells comprising by transformation or transfection an expression vector encoding an OPG fusion polypeptide may be cultured using standard media known to the skilled artisan. The media will usually contain all nutrients necessary for the growth and survival of the cells. Suitable media for culturing *E. coli* cells are for example, Luria Broth (LB) and/or Terrific Broth (TB). Suitable media for culturing eukaryotic cells are RPMI 1640, MEM, DMEM, all of which may be supplemented with serum and/or growth factors as required by the particular cell line being cultured. A suitable medium for insect cultures is Grace's medium supplemented with yeastolate, lactalbumin hydrolysate, and/or fetal calf serum as necessary (Gibco Life Technologies, Gaithersburg, MD).

Typically, an antibiotic or other compound useful for selective growth of transfected or transformed cells is added as a supplement to the media. The compound to be used will be dictated by the selectable marker element present on the plasmid with which the host cell was transformed. For example, where the selectable marker element is kanamycin resistance, the compound added to the culture medium will be kanamycin; where the selectable marker element is ampicillin resistance, the compound added to the culture medium will be ampicillin.

The amount of an OPG fusion polypeptide produced by a host cell can be evaluated using standard methods known in the art. Such methods include, without limitation, Western blot analysis, SDS-polyacrylamide gel electrophoresis, non-denaturing gel electrophoresis, HPLC separation, immunoprecipitation, and/or activity assays such as DNA binding gel shift assays.

Where an OPG fusion polypeptide is prepared without a tag attached, and no antibodies are available, other well known procedures for purification can be used. Such procedures include, without limitation, ion exchange chromatography, molecular sieve chromatography, HPLC, native gel electrophoresis in combination with gel elution, and preparative isoelectric focusing ("Isoprime" machine/technique, Hoefer Scientific). In some cases, two or more of these techniques may be combined to achieve increased purity.

If an OPG fusion polypeptide is produced intracellularly, the intracellular material (including inclusion bodies for gram-negative bacteria) can be extracted from the host cell using any standard technique known to the skilled artisan. For example, the host cells can be lysed to release the contents of the periplasm/cytoplasm by French press, homogenization, and/or sonication followed by centrifugation.

If an OPG fusion polypeptide has formed inclusion bodies in the cytosol, the inclusion bodies can often bind to the inner and/or outer cellular membranes and thus will be found primarily in the pellet material after centrifugation. The pellet material can then be treated at pH extremes or with chaotropic agent such as a detergent, guanidine, guanidine derivatives, urea, or urea derivatives in the presence of a reducing agent such as dithiothreitol at alkaline pH or tris carboxyethyl phosphine at acid pH to release, break apart, and solubilize the inclusion bodies. An OPG fusion polypeptide in its now soluble form can then be analyzed using gel electrophoresis, immunoprecipitation or the like. If it is desired to isolate an OPG polypeptide, isolation may be accomplished using standard methods such as those set forth below and in Marston et al. (Meth. Enz., 182, 264-275 (1990)).

In some cases, an OPG fusion polypeptide may not be biologically active upon isolation. Various methods for "refolding" or converting the polypeptide to its tertiary structure and generating disulfide linkages, can be used to restore biological activity. Such methods include exposing the solubilized polypeptide to a pH usually above 7 and in the presence of a particular concentration of a chaotrope. The selection of chaotrope is very similar to the choices used for inclusion body solubilization, but usually the chaotrope is used at a lower concentration and is not necessarily the same as chaotropes used for the solubilization. In most cases the refolding/oxidation solution will also contain a reducing agent or the reducing agent plus its oxidized form in a specific ratio to generate a particular redox potential allowing for disulfide shuffling to occur in the formation of the protein's cysteine bridge(s). Some of the commonly used redox couples include cysteine/cystamine, glutathione (GSH)/dithiobis GSH, cupric chloride, dithiothreitol(DTT)/dithiane DTT, and 2-mercaptoethanol(βME)/dithio-β(ME). In many instances, a cosolvent may be used or may be needed to increase the efficiency of the refolding and the more common reagents used for this purpose include glycerol, polyethylene glycol of various molecular weights, arginine and the like.

### Derivatives

The present OPG fusion proteins, and variants and fragments thereof, are derivatized by the attachment of one or more chemical moieties. As an example, a fusion of OPG and Fc polypeptide may be derivatized on either OPG or Fc moieties, or both. These chemically modified derivatives may be further formulated for intraarterial, intraperitoneal, intramuscular subcutaneous, intravenous, oral, nasal, pulmonary, topical or other routes of administration as discussed below. Chemical modification of biologically active proteins has been found to provide additional advantages under certain circumstances, such as increasing the stability and circulation time of the therapeutic protein and decreasing immunogenicity. See, U.S. Patent No. 4,179,337. For a review, see Abuchowski et *al.,* in Enzymes as Drugs. (J. S. Holcerberg and J. Roberts, eds. pp. 367-383 (1981)); Francis et *al.,* supra.

The chemical moieties suitable for such derivatization may be selected from among various water soluble polymers. One skilled in the art will be able to select the desired polymer based on such considerations as whether the polymer/protein conjugate will be used therapeutically, and if so, the desired dosage, circulation time, resistance to proteolysis, and other considerations. For the present proteins, the effectiveness of the derivatization may be ascertained by administering the derivative, in the desired form (*i.e.*, by osmotic pump, or, more preferably, by injection or infusion, or, further formulated for oral, pulmonary or nasal delivery, for example), and observing biological effects as described herein.

The water soluble polymer may be selected from the group consisting of, for example, polyethylene glycol, copolymers of ethylene glycol/propylene glycol, carboxymethylcellulose, dextran, polyvinyl alcohol, polyvinyl pyrolidone, poly-1, 3-dioxolane, poly-1,3,6-trioxane, ethylene/maleic anhydride copolymer, polyaminoacids (either homopolymers or random copolymers), and dextran or poly(n-vinyl pyrolidone)polyethylene glycol, propylene glycol homopolymers, polypropylene oxide/ethylene oxide co-polymers, polyoxyethylated polyols and polyvinyl alcohol. Polyethylene glycol propionaldenhyde may have advantages in manufacturing due to its stability in water. Also, succinate and styrene may also be used. In addition, polyaminoacids may be selected from the group consisting of serum album (such as human serum albumin), or other polyaminoacids, e.g. lysines.

The polymer may be of any molecular weight, and may be branched or unbranched. For polyethylene glycol, the preferred molecular weight is between about 2 kDa and about 100 kDa (the term "about" indicating that in preparations of polyethylene glycol, some molecules will weigh more, some less, than the stated molecular weight) for ease in handling and manufacturing.

The number of polymer molecules so attached to an OPG fusion polypeptide may vary, and one skilled in the art will be able to ascertain the effect on function. One may mono-derivatize, or may provide for a di-, tri-, tetra- or some combination of derivatization, with the same or different chemical moieties (*e.g.*, polymers, such as different weights of polyethylene glycols). The proportion of polymer molecules to protein (or peptide) molecules will vary, as will their concentrations in the reaction mixture. In general, the optimum ratio (in terms of efficiency of reaction in that there is no excess unreacted protein or polymer) will be determined by factors such as the desired degree of derivatization (*e.g.*, mono, di-, tri-, etc.), the molecular weight of the polymer selected, whether the polymer is branched or unbranched, and the reaction conditions.

The chemical moieties should be attached to an OPG fusion protein with consideration of effects on functional or antigenic domains of the protein. There are a number of attachment methods available to those skilled in the art (EP 0401384 (coupling PEG to G-CSF); Malik *et al.,* Exp. Hematol. 20, 1028-1035 (1992) (reporting pegylation of GM-CSF using tresyl chloride)). For example, polyethylene glycol may be covalently bound through amino acid residues having a free amino group (e.g., lysine, arginine, or the N-terminal amino acid residue) or a free carboxyl group (e.g., aspartic acid, glutamic acid, and the C-terminal amino acid residue). Amino acid residues having a free sulfhydryl group (e.g., cysteine) may also be used. Preferred for therapeutic purposes is attachment at an amino group, such as attachment at the N-terminus or lysine group. Attachment at residues important for receptor binding should be avoided if receptor binding is desired.

One may specifically desire N-terminally chemically modified OPG fusion protein. Using polyethylene glycol as an example of the chemical moiety, a preparation of substantially N-terminally pegylated OPG fusion polypeptide may be obtained by derivatizing the polypeptide at free amino groups and separating N-terminally pegylated material from a population of pegylated protein molecules. Alternatively, selective N-terminal chemical modification may be accomplished by reductive alkylation which exploits differential reactivity of different types of primary amino groups (lysine versus the N-terminal) available for derivatization in a particular protein. Under the appropriate reaction conditions, substantially selective derivatization of the protein at the N-terminus with a carbonyl group containing polymer is achieved. Polyethylene glycol propionaldehyde, containing a single reactive aldehyde, may be used.

An N-terminally monopegylated derivative is preferred for ease in production of a therapeutic. N-terminal pegylation ensures a homogenous product as characterization of the product is simplified relative to di-, tri- or other multi-pegylated products. The use of the above reductive alkylation process for preparation of an N-terminal product is preferred for ease in commercial manufacturing.

### Uses of the polypeptides

The fusion polypeptides of the invention are used for preparing medicaments for the prevention and/or treatment of loss of bone mass associated with cancer in a mammal, such as prevention and/or treatment of replacement of structurally sound bone with disorganized bone; or in the prevention of metastasis to bone. Bone loss is manifested in a variety of conditions including the following: Hypercalcemia resulting from solid tumors (breast, lung and kidney) and hematologic malignacies (multiple myeloma and Osteolytic metastasis. Replacement of structurally sound bone with disorganized structurally incompetent bone is seen in in osteosclerotic bone metastases.

In an embodiment of the invention, the OPG fusion polypeptides of the invention, by virtue of increased activity and circulating half-life, are advantageously used for preparing medicaments to treat bone loss resulting from osteolytic destruction of bone caused by malignant or metastatic tumors. OPG polypeptides of the invention may be used to treat bone loss associated with breast, prostate, thyroid, kidney, lung, esophogeal, rectal, bladder, cervical, ovarian and liver cancers as well as cancer of the gastrointestional tract. Also included is bone loss associated with certain hematological malignancies such as multiple myeloma.

The OPG fusion proteins of the invention are to be administered alone or in combination with other therapeutic agents, in particular, in combination with other cancer therapy agents. Such agents generally include radiation therapy or chemotherapy. Chemotherapy may involve treatment with one or more of the following: anthracyclines, taxol, tamoxifene, doxorubicin, 5-fluorouracil, and other drugs known to the skilled worker. In one embodiment, the cancer therapy agent is a luteinizing hormone-releasing hormone (LHRH) antagonist comprising the following structure:
A-B-C-D-E-F-G-H-I-J
wherein
A is pyro-glu, Ac-D-Nal, Ac-D-Qal; Ac-Sar, or Ac-D-Pal;
B is His or 4-Cl-D-Phe;
C is Trp, D-Pal, D-Nal, L-Nal-D-Pal(N-O), or D-Trp;
D is Ser;
E is N-Me-Ala, Tyr, N-Me-Tyr, Ser, Lys(iPr), 4-Cl-Phe, His, Asn, Met, Ala, Arg or Ile;
F is 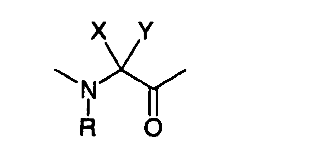 wherein R and X are independently, H and alkyl; and Y comprises a small polar entity.
G is Leu or Trp;
H is Lys(iPr), Gln, Met, or Arg;
I is Pro; and
J is Gly-NH2 or D-Ala-NH2;
or a pharmaceutically acceptable salt thereof.

In another embodiment, an LHRH antagonist comprises the peptide:
N-Ac-D-Nal-4-Cl-Phe-D-Pal-Ser-N-Me-Tyr-D-Asn-Leu-Lys(iPr)-Pro-D-Ala-NH2.

Standard abbreviations and conventions are used herein and the following non-standard residues and moieties are abbreviated as follows:
- Nal: 3-(2-napthyl)alaninyl
- 4-Cl-Phe: (4'-chlorophenyl)alaninyl
- Pal: 3-(3'-pyridyl)alaninyl
- Pal(N-O): 3-(3'-pyridine-N-oxide)alaninyl
- iPr-Lys: N-epsilon-2-propyl-lysinyl
- Qal: 3-(2'-quinolinyl)alaninyl

Alternative forms of LHRH antagonist decapeptides are also encompassed by the invention. Such decapeptides are described in U.S. Patent No. 5,843,901.

Also included are therapeutic antibodies including mouse, mouse-human chimeric, CDR-grafted, humanized or fully human antibodies, or synthetic antibodies such as those selected by screening antibody libraries. Examples of such antibodies include those which bind to cell surface proteins Her2, CDC20, CDC33, mucin-like glycoprotein and epidermal growth factor receptor (EGFR) present on tumor cells and optionally induce a cytostatic and/or cytotoxic effect on tumor cells displaying these proteins. Examples of such antibodies include HERCEPTIN for treatment of breast cancer and RITUXAN for the treatment of non-Hodgkin's lymphoma. Also included as cancer therapy agents are polypeptides which selectively induce apoptosis in tumor cells, such as the TNF-related polypeptide TRAIL. OPG fusion proteins may be administered prior to, concurrent with, or subsequent to treatment with a cancer therapy agent. OPG fusion proteins may be administered prophylactially to prevent or mitigate the onset of bone loss by metastatic cancer or may be given for the treatment of an existing condition of bone loss due to metastasis.

OPG fusion polypeptides of the invention may be used to prepare medicaments to prevent and/or treat bone loss associated with multiple myeloma or to prevent and/or treat the disease itself. Multiple myeloma is a B cell derived tumor that results in significant morbidity and mortality. The most striking common clinical manifestation is the focal bone loss due to increased osteoclast activation in localized regions. The majority of myeloma patients (~95%) usually present with destructive bone lesions visible by radiological analysis, and suffer from extreme, intractable skeletal pain. Patients with myeloma are particularly susceptible to pathological fractures of involved bone, which occur either spontaneously or due to trivial injury. The skeletal lesions that occur during myeloma not only lead to bone fractures, but also deformity and occasionally nerve compression, particularly in the vertebral spine. In some patients, a pathological increase in serum calcium (hypercalcemia) occurs, and can cause significant problems during disease treatment. OPG may be administered to patients to block bone resorption and release of calcium, thereby reducing the risk of fractures and spinal deformity.

Myeloma cells do not directly participate in bone destruction, but instead produce extracellular signals that lead to osteoclast differentiation and activation. Osteoclasts in turn produce the highest levels of the potent cytokine IL-6 of any cell type in the body, particularly when they become activated. IL-6 is a B-cell growth factor, and is required for the growth of both murine and human myeloma cells in vitro. A TNF-related protein OPG ligand (OPGL) is responsible for inducing osteoclast differentiation and activation (See WO98/46751). Myeloma cells may either directly or indirectly produce this factor to osteoclasts, resulting in local bone lysis surrounding the myeloma cells embedded in bone marrow spaces. The normal osteoclasts adjacent to the myeloma cell in turn produce IL-6, leading to local expansion of the tumor cells. Myeloma cells expand in a clonal fashion and occupy bone spaces that are being created by inappropriate bone resorption.

It has been observed that OPG administration in rodents induces rapid death of the osteoclast population. A reduction in osteoclasts could eliminate the increase in IL-6 production by osteoclasts and affect the growth and survival of myeloma cells within trabecular bone. Thus, OPG treatment in myeloma patients would not only block the hyper resorption of bone, but could also affect the expansion and survival of the tumor itself. B-cells are known to express the receptor for OPGL, referred to as osteoclast differentiation and activation receptor, or ODAR. Myeloma cells also express ODAR, and in addition may produce OPGL. The expression of both OPGL and ODAR on the same cell population may create an autocrine stimulus that affects survival of the myeloma cell. Thus, OPG treatment could directly affect tumor cell survival, thus decreasing or eliminating, the tumor burden seen in myeloma patients.

### Pharmaceutical Compositions

The use according to the present invention also provides for pharmaceutical compositions comprising OPG fusion proteins, and variants, fragments and derivatives thereof. Such pharmaceutical compositions may be for administration for injection, or for oral, pulmonary, nasal, transdermal or other forms of administration. In general, the use according to the invention results in pharmaceutical compositions comprising effective amounts of an OPG fusion protein together with pharmaceutically acceptable diluents, preservatives, solubilizers, emulsifiers, adjuvants and/or carriers. An effective or a therapeutically effective amount of an OPG fusion protein is an amount sufficient to reduce the rate and/or extent of bone loss as determined by assays and procedures described herein.

Pharmaceutical compositions of the invention include diluents of various buffer content (*e.g*., Tris-HCl, acetate, phosphate), pH and ionic strength; additives such as detergents and solubilizing agents (*e.g.*, Tween 80, Polysorbate 80), anti-oxidants (*e.g.*, ascorbic acid, sodium metabisulfite), preservatives (*e.g.*, Thimersol, benzyl alcohol) and bulking substances (*e.g.*, lactose, mannitol); incorporation of the material into particulate preparations of polymeric compounds such as polylactic acid, polyglycolic acid, etc. or into liposomes. Hylauronic acid may also be used, and this may have the effect of promoting sustained duration in the circulation. Such compositions may influence the physical state, stability, rate of in vivo release, and rate of in vivo clearance of the present proteins and derivatives. See, *e.g.*, Remington's Pharmaceutical Sciences, 18th Ed. (1990, Mack Publishing Co., Easton, PA 18042) pages 1435-1712. The compositions may be prepared in liquid form, or may be in dried powder, such as lyophilized form. Implantable sustained release formulations are also contemplated, as are transdermal formulations.

Contemplated for use herein are oral solid dosage forms, which are described generally in Remington's Pharmaceutical Sciences, 18th Ed. 1990 (Mack Publishing Co. Easton PA 18042) at Chapter 89. Solid dosage forms include tablets, capsules, pills, troches or lozenges, cachets or pellets. Also, liposomal or proteinoid encapsulation may be used to formulate the present compositions (as, for example, proteinoid microspheres reported in U.S. Patent No. 4,925,673). Liposomal encapsulation may be used and the liposomes may be derivatized with various polymers (*e.g.,* U.S. Patent No. 5,013,556). A description of possible solid dosage forms for the therapeutic is given by Marshall, K. In: *Modern Pharmaceutics* Edited by G. S. Banker and C. T. Rhodes Chapter 10, 1979. In general, the formulation will include an OPG fusion protein, or a variant, fragment or derivative thereof, and inert ingredients which allow for protection against the stomach environment, and release of the biologically active material in the intestine.

An OPG fusion protein may optionally be chemically modified so that oral delivery of the derivative is efficacious. Generally, the chemical modification contemplated is the attachment of at least one moiety to the protein (or peptide) molecule itself, where said moiety permits (a) inhibition of proteolysis; and (b) uptake into the blood stream from the stomach or intestine. Also desired is the increase in overall stability of the protein and increase in circulation time in the body. Examples of such moieties include polyethylene glycol, copolymers of ethylene glycol and propylene glycol, carboxymethyl cellulose, dextran, polyvinyl alcohol, polyvinyl pyrrolidone and polyproline. Abuchowski and Davis, Soluble Polymer-Enzyme Adducts. In: "Enzymes as Drugs", Hocenberg and Roberts, eds., Wiley-Interscience, New York, NY, (1981), pp 367-383; Newmark, *et al.,* J. Appl. Biochem. 4: 185-189 (1982). Other polymers that could be used are poly-1,3-dioxolane and poly-1,3,6-tioxocane. Preferred for pharmaceutical usage, as indicated above, are polyethylene glycol moieties.

To ensure resistance to degradation in the stomach following oral administration, a coating impermeable to at least pH 5.0 is essential. Examples of the more common inert ingredients that are used as enteric coatings for oral formulations are cellulose acetate trimellitate (CAT), hydroxypropylmethyl cellulose phthalate (HPMCP), HPMCP 50, HPMCP 55, polyvinyl acetate phthalate (PVAP), Eudragit L30D, Aquateric, cellulose acetate phthalate (CAP), Eudragit L, Eudragit S, and Shellac. These coatings may be used as mixed films.

An OPG fusion protein may be included in a formulation as fine multiparticulates in the form of granules or pellets of particle size about 1 mm. The formulation of the material for capsule administration could also be as a powder, lightly compressed plugs or even as tablets.

Pharmaceutical compositions of the invention include diluents such as carbohydrates, especially mannitol, α-lactose, anhydrous lactose, cellulose, sucrose, modified dextrans and starch. Certain inorganic salts may be also be used as fillers including calcium triphosphate, magnesium carbonate and sodium chloride. Some commercially available diluents are Fast-Flo, Emdex, STA-Rx 1500, Emcompress and Avicell.

Disintegrants may be included in solid dosage formulations. Materials used as disintegrates include but are not limited to starch including the commercial disintegrant based on starch, Explotab. Sodium starch glycolate, Amberlite, sodium carboxymethylcellulose, ultramylopectin, sodium alginate, gelatin, orange peel, acid carboxymethyl cellulose, natural sponge and bentonite may all be used. Another form of the disintegrants are the insoluble cationic exchange resins. Powdered gums may be used as disintegrants and as binders and these can include powdered gums such as agar, Karaya or tragacanth. Alginic acid and its sodium salt are also useful as disintegrants.

Binders may be used to form hard tablets and include materials from natural products such as acacia, tragacanth, starch and gelatin. Others include methyl cellulose (MC), ethyl cellulose (EC) and carboxymethyl cellulose (CMC). Polyvinyl pyrrolidone (PVP) and hydroxypropylmethyl cellulose (HPMC) could both be used in alcoholic solutions to granulate the therapeutic.

Lubricants that may be added to a formulation include but are not limited to; stearic acid including its magnesium and calcium salts, polytetrafluoroethylene (PTFE), liquid paraffin, vegetable oils and waxes. Soluble lubricants may also be used such as sodium lauryl sulfate, magnesium lauryl sulfate, polyethylene glycol of various molecular weights, Carbowax 4000 and 6000.

Glidants that might improve the flow properties of the drug during formulation and to aid rearrangement during compression might be added. The glidants may include starch, talc, pyrogenic silica and hydrated silicoaluminate.

To aid dissolution of an OPG fusion protein composition, a surfactant might be added as a wetting agent. Surfactants may include anionic detergents such as sodium lauryl sulfate, dioctyl sodium sulfosuccinate and dioctyl sodium sulfonate. Cationic detergents might be used and could include benzalkonium chloride or benzethomium chloride. The list of potential nonionic detergents that could be included in the formulation as surfactants are lauromacrogol 400, polyoxyl 40 stearate, polyoxyethylene hydrogenated castor oil 10, 50 and 60, glycerol monostearate, polysorbate 40, 60, 65 and 80, sucrose fatty acid ester, methyl cellulose and carboxymethyl cellulose. These surfactants could be present in the formulation of the protein or derivative either alone or as a mixture in different ratios.

Additives which potentially enhance uptake of a protein are, for instance, the fatty acids oleic acid, linoleic acid and linolenic acid.

A controlled release formulation may be desirable. An OPG fusion protein may be incorporated into an inert matrix which permits release by either diffusion or leaching mechanisms *e.g.*, gums. Slowly degenerating matrices may also be incorporated into the formulation, *e.g.*, alginates, polysaccahrides. Another form of a controlled release of this therapeutic is by a method based on the Oros therapeutic system (Alza Corp.), *i.e.*, the drug is enclosed in a semipermeable membrane which allows water to enter and push drug out through a single small opening due to osmotic effects. Some enteric coatings also have a delayed release effect.

Other coatings may be used in a formulation. For example, a film coated tablet may comprise materials from two different groups. The first group includes nonenteric materials such as methyl cellulose, ethyl cellulose, hydroxyethyl cellulose, methylhydroxyethyl cellulose, hydroxypropyl cellulose, hydroxypropyl methyl cellulose, sodium carboxy-methyl cellulose, providone and the polyethylene glycols. The second group consists of the enteric materials that are commonly esters of phthalic acid. A mix of materials might be used to provide the optimum film coating. Film coating may be carried out in a pan coater or in a fluidized bed or by compression coating.

Also contemplated herein is pulmonary delivery of an OPG polypeptide or fusion protein. The protein is delivered to the lungs of a mammal while inhaling and traverses across the lung epithelial lining to the blood stream. (Other reports of this include Adjei *et al*., Pharmaceutical Research 7: 565-569 (1990); Adjei *et al*., International Journal of Pharmaceutics 63: 135-144 (1990)(leuprolide acetate); Braquet *et al.,* Journal of Cardiovascular Pharmacology 13 (suppl. 5): s.143-146 (1989)(endothelin-1); Hubbard *et al*., Annals of Internal Medicine 3: 206-212 (1989)(α1-antitrypsin); Smith *et al.,* J. Clin. Invest. 84: 1145-1146 (1989)(α1-proteinase); Oswein *et al*., "Aerosolization of Proteins", Proceedings of Symposium on Respiratory Drug Delivery II, Keystone, Colorado, March, 1990 (recombinant human growth hormone); Debs *et al.,* The Journal of Immunology 140: 3482-3488 (1988)(interferon a and tumor necrosis factor α) and U.S. Patent No. 5,284,656 (granulocyte colony stimulating factor).

Contemplated for use in the practice of this invention are a wide range of mechanical devices designed for pulmonary delivery of therapeutic products, including but not limited to nebulizers, metered dose inhalers, and powder inhalers, all of which are familiar to those skilled in the art. Some specific examples of commercially available devices suitable for the practice of this invention are the Ultravent nebulizer, manufactured by Mallinckrodt, Inc., St. Louis, Missouri; the Acorn II nebulizer, manufactured by Marquest Medical Products, Englewood, Colorado; the Ventolin metered dose inhaler, manufactured by Glaxo Inc., Research Triangle Park, North Carolina; and the Spinhaler powder inhaler, manufactured by Fisons Corp., Bedford, Massachusetts.

All such devices require the use of formulations suitable for the dispensing of an OPG fusion protein, or a variant, fragment or derivative thereof. Typically, each formulation is specific to the type of device employed and may involve the use of an appropriate propellant material, in addition to diluents, adjuvants and/or carriers useful in therapy.

An OPG fusion protein should most advantageously be prepared in particulate form with an average particle size of less than 10 µm (or microns), most preferably 0.5 to 5 µm, for most effective delivery to the distal lung.

Carriers include carbohydrates such as trehalose, mannitol, xylitol, sucrose, lactose, and sorbitol. Other ingredients for use in formulations may include DPPC, DOPE, DSPC and DOPC. Natural or synthetic surfactants may be used. Polyethylene glycol may be used (even apart from its use in derivatizing the protein or analog). Dextrans, such as cyclodextran, may be used. Bile salts and other related enhancers may be used. Cellulose and cellulose derivatives may be used. Amino acids may be used, such as use in a buffer formulation. The use of liposomes, microcapsules or microspheres, inclusion complexes, or other types of carriers is also contemplated.

Nasal delivery of an OPG fusion protein is also contemplated. Nasal delivery allows the passage of the protein to the blood stream directly after administering the therapeutic product to the nose, without the necessity for deposition of the product in the lung. Formulations for nasal delivery include those with dextran or cyclodextran. Delivery via transport across other mucus membranes is also contemplated.

### Dosages

OPG fusion polypeptides are to be administered in a therapeutically effective amount to prevent and/or treat loss of bone associated with metastatic bone disease. A "therapeutically effective amount" of an OPG fusion polypeptide is that amount which reduces the loss of bone mass. Bone mass is measured by a variety of known methods such as single photon absorptiometry (SPA), dual photon absorptiomerty (DPA), dual energy X-ray absorptiometry (DEXA), quantitative computed tomography (QCT), and ultrasonography (See Johnston et al. in Primer on the Metabolic Bone Disease and Disorders of Mineral Metabolism, 2^{nd} ed., M.J. Favrus, ed. Raven Press pp. 237-146)). One skilled in the art can use these methods to determine a therapeutically effective amount of an OPG fusion polypeptide. A therapeutically effective amount may also be determined by measuring changes in biochemical markers for bone turnover, such as serum osteocalcin, serum alkaline phosphatase, serum procollagen I extension peptides, urinary or serum C-terminal or N-terminal telopeptide of collagen, urinary calcium, hydroxyproline and urinary pyridinoline and deoxypyridinoline. It is generally recognized that a decrease in the levels of the aforementioned biochemical markers indicates that bone resorption is decreased and bone loss is being reduced. Alternatively, a therapeutically effective amount of an OPG fusion polypeptide may also be determined by measuring an increase in bone strength, in particular an increase in torsional (twisting) strength of bone.

In general, a therapeutically effective amount of an OPG fusion polypeptide is from about 0.1 mg/kg to about 10 mg/kg, preferably from about 1mg/kg to about 10 mg/kg. By virtue of the increased half-life of an OPG fusion polypeptide, and especially a fusion of OPG to an immunoglobulin Fc region, administration will be less frequent than for an unmodified OPG polypeptide. The frequency of administration may be about one time per month, or alternatively, one time every two months, or one time every three months. It will be appreciated by one skilled in the art that the exact dosage and frequency of administration will depend upon several factors, including formulation, route of administration, condition being treated, and so forth, and may be readily determined by the skilled worker.

The amount of OPG fusion protein which has been administered may be determined using diagnostic assays for the fusion protein. Such diagnostic assays may be in the form of an antibody assay, such as an antibody sandwich assay, wherein the antibody specifically binds to the OPG fusion protein, but does not bind to endogenous, naturally circulating OPG or to a form of the heterologous protein fused to OPG which may also circulate naturally, such as an Fc region of a naturally occurring antibody. Antibody based assays for determining OPG fusion protein levels may be carried out in a variety of formats that are known to one skilled in the art.

The following examples are offered to more fully illustrate the invention, but are not construed as limiting the scope thereof.

### EXAMPLE 1

### Construction and Expression of OPG Fusion Polypeptides

Plasmids encoding OPG[1-194]-Fc, OPG[1-201]-Fc, OPG[1-194]-FcΔC, OPG[1-201]-FcΔC, OPG[1-194]-FcG₁₀, and metFcΔC-OPG[22-194] for use in producing the corresponding OPG fusion polypeptides are constructed generally as described in WO97/23614. The polypeptide sequences are shown in Figures 3-8, respectively.

Expression of an OPG fusion polypeptide in mammalian and bacterial host cells was carried out generally as described in WO97/23614.

### EXAMPLE 2

### OPG Activity in a Breast Cancer Model for Lytic Bone Disease

Female Balb/c nu/nu mice aged 7-8 weeks were injected with human MDA-MB-231 breast cancer cells (1.0 x 10⁵ cells/mouse; ATCC accession no. HTB-26) directly into the systemic circulation via the left ventricle. Immediately following tumor inoculation, the mice were treated by intravenous injection with either phosphate buffered saline (PBS) or met FcΔC-OPG[22-194] (25mg/kg) three times per week for 4 weeks. The number of lesions/mouse was assessed from radiographs. Bone, heart, lung, liver, kidneys, adrenals, ovaries, brain, pancreas and spleen were evaluated histologically for the presence of tumor foci as described below.

As shown in Figure 9, radiographic lesions are apparent in the long bones 4 weeks after inoculation with MDA-MB-231 cells. The associated bone destruction is completely inhibited by intravenous administration of met FcΔC-OPG[22-194] at a dose of 25mg/kg three times per week commencing at the time of inoculation (6.2 ± 0.8 vs. 0.0 ± 0.0 lesions/mouse, p < 0.001).

### EXAMPLE 3

### OPG Activity in a Mouse Adenocarcinoma Model for Lytic Bone Disease

Female CDF1 mice aged 7-8 weeks were injected with murine C26-DCT adenocarcinoma cells (obtained from the Tumor Repository of the National Cancer Institute; 1.0 x 10⁵ cells/mouse) directly into the systemic circulation via the left ventricle. Immediately following tumor inoculation, mice were treated by intravenous injection with either PBS or met FcΔC-OPG[22-194] (25mg/kg) every 3 days for 9 days. On day 10 the mice were radiographed and tissues were sampled for histological evaluation.

As shown in Figure 9, radiographic lesions are evident 10 days after intra-cardiac inoculation of C26-DCT cells (3.1 ± 0.6 lesions/mouse) and bone destruction is inhibited by intravenous injection of FcdC-OPG[22-194].

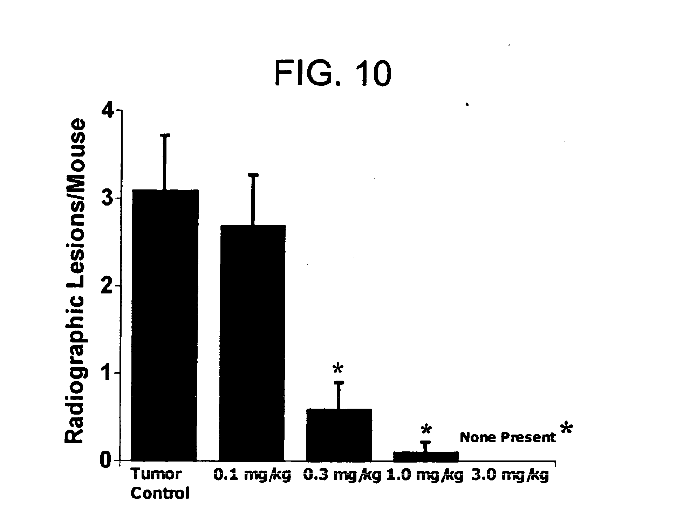

In an additional study, mice were inoculated with C26-DCT cells, as above, and treated by intravenous injection with either PBS or met Fc-OPG[22-194] at 3, 1, 0.3, or 0.1mg/kg every 3 days for 9 days. On day 10 the mice were radiographed. Radiographs were assessed and tissues were handled as described below. Radiographic lesions were reduced in a dose dependent manner by intravenous met FcΔC-OPG [22-194] treatment. (Figure 10)

### EXAMPLE 4

### OPG Activity in a Mouse Adenocarcinoma Model for Hypercalcemia

Male Balb/c x DBA/2 (CDF1) mice at 10-12 weeks of age were purchased from either Harlan Sprague Dawley (San Diego, CA) or Charles River (Wilmington, MA) .

The C-26 tumor, originally induced in a female Balb/c mouse by repeated intrarectal instillation of *N*-nitroso-*N*-methylurethan (NMU) (Corbett et al. Cancer Res. 35, 2434-2439 (1975)), was obtained from the Tumor Repository of the National Cancer Institute in the form of tissue fragments. The fragments were mechanically disrupted and placed into culture under standard tissue culture conditions (37°C, 5-6% CO₂), which gave rise to an adherent cell line that could be continually propagated and was subsequently frozen down as such. The media employed for passage of the cells in vitro is DMEM plus 10% FCS, 1x pen-strep/glutamine and 1x nonessential amino acids. A separate frozen vial of cells from a common stock was used for the initiation of all experiments. After initial reestablishment in culture, cells were harvested by trypsinization, followed by several washes and resuspension in DMEM with no additives to a concentration of 2.5 x 10' cells/ml. Animals were injected subcutaneously (SQ) over a shaved area of the right flank with 0.2 cc (0.5 x 10⁶ cells). Under these conditions tumor development was found to be very consistent with minimal variability.

Treatment began on either day 8 (prevention studies) or when blood ionized calcium levels reached a level >1.60 mmol/L (treatment studies). Treatment lasted for 8 days in the prevention studies and for 4 days in the treatment studies. In the prevention studies metFcΔC-OPG[22-1941] was administered daily in a PBS vehicle as a subcutaneous injection in the flank. In the treatment studies, met FcΔC-OPG[22-194] was administered as a single intravenous injection in PBS vehicle. In both studies normal and tumor bearing control animals received a similar injection of PBS.

During the course of the study, mice were weighed daily (while on treatment), and blood ionized calcium levels were monitored either every other day in the prevention studies (prior to the drug administration on that day) or daily in the treatment studies. Blood was sampled retro-orbitably and blood ionized calcium determinations were performed using a blood ionized calcium/pH analyzer (Chiron Diagnostics #634, Norwood, MA).

### Histological Evaluation

One leg from each animal (femur and tibia connected) was harvested at the conclusion of the study. The bones were fixed in phosphate buffered zinc formalin, decalcified in formic acid and embedded in paraffin. Sections were taken through the midregion of the distal femur and proximal tibia, reacted to demonstrate tartrate resistant acid phosphatase activity (TRAP), and counterstained with hematoxylin. In this staining procedure osteoclasts are stained red, while other cell types, bone and cartilage are stained various shades of blue.

Measurements were made of the area just distal to the proximal tibial growth plate (in the area of the primary spongiosa), and in an area of the cortical shaft of the tibia 2mm distal to the first measurement area using an Osteomeasure bone analysis program (Osteometrics Inc., Decatur, GA). Two separate regions of the tibia were chosen for measurement so an accurate determination of the tumor induced increase in bone resorption could be obtained. The field of measurement consisted of a 1mm x 1mm square area in both locations and did not include the growth plate in the first region. The parameters measured were the number and active surface of osteoclasts, and bone surface. Results were recorded as osteoclast number (OcN), osteoclast perimeter (active osteoclast surface) (OcPm), bone perimeter (bone surface) (BPm). In order for an osteoclast to be considered for measurement, it had to be TRAP positive and in contact with a bone surface. The active osteoclast surface was the part of the osteoclast that was in direct contact with a bone surface. All measurements were made by tracing the section image onto a digitizing platen with the aid of a camera lucida attachment on the microscope.

### Results

OPG treatment moderated the effects of the C-26 adenocarcinoma on body weight loss. Mice receiving a daily 2.5mg/kg dose of OPG lost an average of 5.2 grams body weight while untreated tumor bearing animals lost an average of 6.2 grams. PBS tumor bearing mice had tumors that were 3.47 ± 0.72% vs OPG 2.5 mg/kg 3.42 ± 0.82%. Weights are: PBS 0.75 ± 0.14g and OPG 2.5: 0.79 ± 0.16g.

### OPG prevents and reverses C-26 tumor induced increases in blood ionized calcium levels

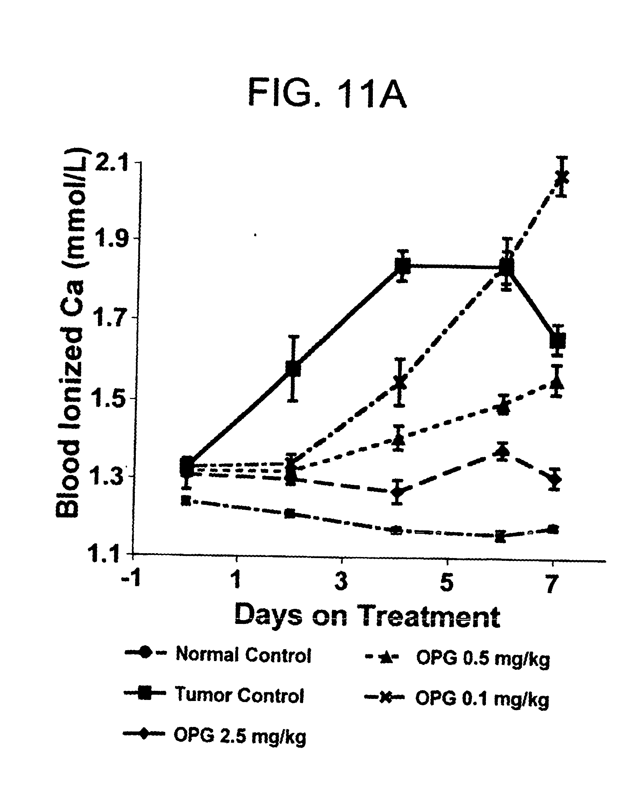

When treatment was commenced on day 9 following implantation of the tumor, OPG dose-dependently inhibited the increase in whole blood ionized calcium levels induced by the tumor (see Figure 11A). Prior to commencing OPG treatment, the tumor bearing mice had slightly increased whole blood ionized calcium levels (1.34 ± 0.06 mmol/L vs 1.25 ± 0.02 mmol/L). In the vehicle treated groups these levels continued to increase throughout the experiment with maximum levels of 1.84 ± 0.12 mmol/L and reached on days 13 and 15, and decreasing slightly by day 16. The OPG treated groups demonstrated a dose dependent inhibition of this increase in whole blood ionized calcium levels throughout the treatment period, with ionized calcium levels reaching a maximum of 1.38 ± 0.06 mmol/L on day15 in the 2.5 mg/kg group. This level of calcium was moderately but significantly higher than that found in non-tumor bearing control animals. OPG treatment had no effect on calcium levels in non-tumor bearing mice.

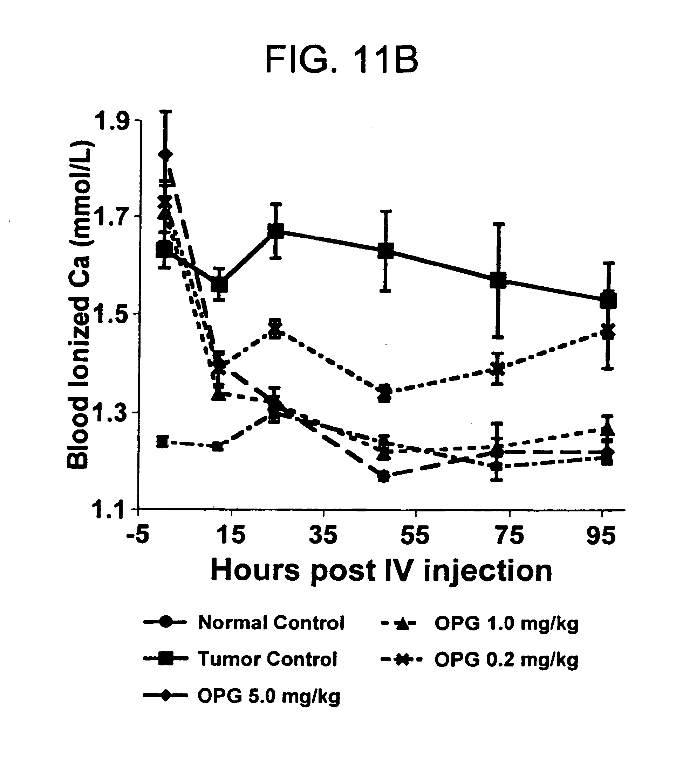

When mice were allowed to become frankly hypercalcemic prior to treatment, a single 5.0 mg/kg dose of OPG produced a rapid decrease in calcium levels with a significant decrease evident by 12 hours and normocalcemia achieved within 24 hours of treatment. (see Figure 11B). OPG maintained calcium levels in the normal range for the remainder of the experiment.

### OPG prevents and reverses C-26 tumor induced increases in osteoclast lined bone surfaces and osteoclast numbers

Osteoclast lined surfaces and osteoclast numbers were markedly elevated in hypercalcemic mice bearing C26 tumors. Treatment with a 2.5 mg/kg dose of OPG either prior to or after the development of hypercalcemia caused almost a completed disappearance of osteoclasts. Osteoclast surface measurements, an indication of the amount of bone resorption, were significantly increased in the tumor bearing animals, 8.95 ± 2.10%, compared to non tumor bearing controls 3.91 ± 1.10%. A 2.5 mg/kg dose of OPG given daily prior to the development of hypercalcemia dose dependently reduced these to 0.13 ± 0.07% which is significantly lower than even the non tumor bearing control animals.

The number of osteoclasts per mm bone surface were also elevated in the tumor bearing animals to 4.41 ± 1.03 /mm compared to non tumor bearing controls 2.00 ± 0.52 /mm. A daily 2.5 mg/kg dose of OPG dose dependently reduced the number of osteoclasts per mm to 0.12 ± 0.06 /mm which is significantly lower then even the non-tumor bearing animals.

### OPG treatment dose dependently decreases the size and number of osteoclasts

Osteoclast surface as a percent of bone surface measurements as well as the number of osteoclasts per mm bone surface were both significantly elevated in the tumor bearing control animals 8.95 ± 1.64 % and 4.12 ± 0.72 osteoclasts per mm respectively, when compared to normal non-tumor bearing animals 3.66 ± 1.01 % and 1.83 ± 0.54 osteoclasts per mm. A daily 2.5 mg/kg dose of OPG significantly reduced these values below even the normal range to 1.26 ± 0.93% and 0.73 ± 0.55 osteoclasts per mm.

## Claims

1. Use of an OPG polypeptide for the preparation of a medicament for the prevention or treatment of bone loss associated with cancer in a mammal, wherein the cancer is selected from the group consisting of breast cancer, prostate cancer, thyroid cancer, cancer of the kidney, lung cancer, esophageal cancer, rectal cancer, bladder cancer, cervical cancer, ovarian cancer, liver cancer, cancer of the gastrointestinal tract, multiple myeloma and colon adenocarcinoma.

2. The use according to claim 1, wherein the OPG polypeptide is to be administered prior to, concurrent with, or subsequent to administration of a cancer therapy agent, and wherein the cancer therapy agent is selected from the group consisting of radiation, chemotherapy, antibodies, or non-antibody polypeptides.

3. The use according to claim 1 or 2, wherein the OPG polypeptide comprises an amino acid sequence as shown in SEQ ID NO:2 or a truncated polypeptide thereof.

4. The use according to claim 3, wherein the OPG polypeptide comprises a carboxy terminal truncation of part or all of amino acid residues 186 - 401 of the sequence as shown in SEQ ID NO:2.

5. The use according to claim 3, wherein the OPG polypeptide comprises amino acid residues 22-194 inclusive as shown in SEQ ID NO:2.

6. The use according to any one of claims 3 to 5, wherein the OPG polypeptide is an OPG fusion polypeptide.

7. The use according to claim 6, wherein the OPG fusion polypeptide comprises a fusion of an Fc region to the N-terminal or C-terminal end of the OPG polypeptide.

8. The use according to claim 7, wherein the OPG fusion polypeptide comprises an Fc region fused to amino acid residues 22-194 of SEQ ID NO:2.

9. The use according to claim 8, wherein the OPG fusion polypeptide consists of the amino acid sequence as shown in SEQ ID NO:5 or SEQ ID NO: 8.

10. The use according to claim 2, wherein chemotherapy comprises anthacyclines, taxol, tamoxifene, doxorubicin, and 5-fluorouracil.

11. The use according to claim 2, wherein the antibodies bind to Her2, CDC20, CDC33, mucin-like glycoprotein, or epidermal growth factor receptor (EGFR) on the surface of tumor cells.

12. The use according to claim 2, wherein the cancer therapy agent comprises a luteinizing hormone-releasing hormone (LHRH) antagonist of the following structure:
A-B-C-D-E-F-G-H-I-J
wherein
A is pyro-glu, Ac-D-Nal, Ac-D-Qal, Ac-Sar, or Ac-D-Pal;
B is His or 4-Cl-D-Phe;
C is Trp, D-Paf, D-Nal, L-Nal-D-Pal(N-0), or D-Trp;
D is Ser,
E is N-Me-Ala, Tyr, N-Me-Tyr, Ser, Lys(iPr), 4-Cl-Phe, His, Asn, Met, Ala, Arg or lle;
F is 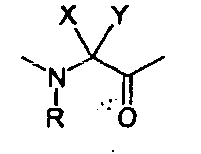 wherein R and X are independently H and alkyl, and Y comprises a small polar entity;
G is Leu or Trp.
H is Lys(iPr), Gln, Met, or Arg;
I is Pro; and
J is Gly-NH₂ or D-Ala-NH₂.

13. The use according to claim 12, wherein the LHRH antagonist comprises the peptide: N-Ac-D-Nal-4-Cl-Phe-D-Pal-Ser-N-Me-Tyr-D-Asn-Leu-Lys(iPr)-Pro-D-Ala-NH2.

14. The use according to claim 1 or 7, wherein the therapeutically effective amount of an OPG polypeptide or an OPG fusion polypeptide is between 0.1 mg/kg and 10 mg/kg.

15. Use of an OPG polypeptide for the preparation of a medicament for the prevention or treatment of multiple myeloma.

## Patentansprüche

1. Verwendung eines OPG-Polypeptids zur Herstellung eines Medikaments zur Vorbeugung oder zur Behandlung von mit Krebs assoziiertem Knochenschwund in einem Säugetier, wobei der Krebs aus der Gruppe ausgewählt ist, die aus Brustkrebs, Prostatakrebs, Schilddrüsenkrebs, Nierenkrebs, Lungenkrebs, Speiseröhrenkrebs, Rektalkrebs, Blasenkrebs, Krebs des Gebärmutterhalses, Eierstockkrebs, Leberkrebs, Krebs des Magen-Darm-Traktes, multiplem Myelom und Adenokarzinom des Dickdarms besteht.

2. Verwendung nach Anspruch 1, wobei das OPG-Polypeptid zur Verabreichung vor, gleichzeitig mit oder nach der Verabreichung eines Krebstherapiemittels vorgesehen ist, und wobei das Krebstherapiemittel aus der Gruppe ausgewählt ist, die aus Bestrahlung, Chemotherapie, Antikörpern oder nicht-Antikörper Polypeptiden besteht.

3. Verwendung nach Anspruch 1 oder 2, wobei das OPG-Polypeptid eine Aminosäurensequenz wie in SEQ ID NO:2 gezeigt oder ein verkürztes Polypeptid davon umfasst.

4. Verwendung nach Anspruch 3, wobei das OPG-Polypeptid eine carboxyterminale Verkürzung eines Teils der Aminosäurenreste 186-401 oder aller Aminosäurenresten 186-401 der Sequenz wie in SEQ ID NO:2 gezeigt umfasst.

5. Verwendung nach Anspruch 3, wobei das OPG-Polypeptid die Aminosäurenreste 22 bis einschließlich 194 wie in SEQ ID NO:2 gezeigt umfasst.

6. Verwendung nach einem der Ansprüche 3 bis 5, wobei das OPG-Polypeptid ein OPG-Fusionspolypeptid ist.

7. Verwendung nach Anspruch 6, wobei das OPG-Fusionspolypeptid eine Fusion einer Fc-Region mit dem N-terminalen oder C-terminalen Ende des OPG-Polypeptids umfasst.

8. Verwendung nach Anspruch 7, wobei das OPG-Fusionspolypeptid eine Fc-Region umfasst, die mit den Aminosäurenresten 22-194 der SEQ ID NO:2 fusioniert ist.

9. Verwendung nach Anspruch 8, wobei das OPG-Fusionspolypeptid aus der Aminosäurensequenz wie in SEQ ID NO:5 oder SEQ ID NO:8 gezeigt besteht.

10. Verwendung nach Anspruch 2, wobei Chemotherapie Anthracycline, Taxol, Tamoxifen, Doxorubicin und 5-Fluoruracil umfasst.

11. Verwendung nach Anspruch 2, wobei die Antikörper an Her2, CDC20, CDC33, mucinähnliches Glycoprotein oder epidermalen Wachstumsfaktor-Rezeptor (EGFR) auf der Oberfläche von Tumorzellen binden.

12. Verwendung nach Anspruch 2, wobei das Krebstherapiemittel ein luteinisierendes Hormon-freisetzendes Hormon (englisch: luteinizing hormone-releasing hormone) (LHRH)-Antagonist der folgenden Struktur umfasst:
A-B-C-D-E-F-G-H-I-J
wobei
A pyro-glu, Ac-D-Nal, Ac-D-Qal, Ac-Sar oder Ac-D-Pal ist;
B His oder 4-Cl-D-Phe ist;
C Trp, D-Paf, D-Nal, L-Nal-D-Pal(N-O) oder D-Trp ist;
D Ser ist;
E N-Me-Ala, Tyr, N-Me-Tyr, Ser, Lys(iPr), 4-Cl-Phe, His, Asn, Met, Ala, Arg oder Ile ist;
F 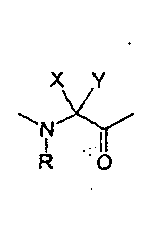 ist wobei R und X unabhängig voneinander H und Alkyl sind, und Y eine kleine polare Gruppe umfasst;
G Leu oder Trp ist;
H Lys(iPr), Gln, Met oder Arg ist;
I Pro ist; und
J Gly-NH₂ oder D-Ala-NH₂ ist.

13. Verwendung nach Anspruch 12, wobei der LHRH-Antagonist das Peptid:
N-Ac-D-Nal-4-Cl-Phe-D-Pal-Ser-N-Me-Tyr-D-Asn-Leu-Lys(iPr)-Pro-D-Ala-NH₂
umfasst.

14. Verwendung nach Anspruch 1 oder 7, wobei die therapeutisch wirksame Menge eines OPG-Polypeptids oder eines OPG-Fusionspolypeptids zwischen 0,1 mg/kg und 10 mg/kg beträgt.

15. Verwendung eines OPG-Polypeptids zur Herstellung eines Medikaments zur Vorbeugung oder zur Behandlung von multiplem Myelom.

## Revendications

1. Utilisation d'un polypeptide de l'OPG pour la préparation d'un médicament destiné à la prévention ou au traitement d'une perte osseuse associée à un cancer chez un mammifère, où le cancer est choisi dans le groupe constitué d'un cancer du sein, d'un cancer de la prostate, d'un cancer de la thyroïde, d'un cancer du rein, d'un cancer du poumon, d'un cancer de 1'oesophage, d'un cancer rectal, d'un cancer de la vessie, d'un cancer cervical, d'un cancer ovarien, d'un cancer du foie, d'un cancer gastro-intestinal, d'un myélome multiple et d'un adénocarcinome du côlon.

2. Utilisation selon la revendication 1, dans laquelle le polypeptide de l'OPG doit être administré avant, en même temps, ou après l'administration d'un agent pour la thérapie d'un cancer, et où l'agent pour la thérapie d'un cancer est choisi dans le groupe constitué de radiations, d'une chimiothérapie, d'anticorps ou de polypeptides qui ne sont pas des anticorps.

3. Utilisation selon la revendication 1 ou 2, dans laquelle le polypeptide de l'OPG est composé d'une séquence d'acides aminés telle que représentée par SEQ ID : 2 ou d'un polypeptide tronqué de celui-ci.

4. Utilisation selon la revendication 3, dans laquelle le polypeptide de l'OPG comprend une troncation carboxy-terminale d'une partie ou de la totalité des résidus d'acides aminés 186 à 401 de la séquence telle que représentée par SEQ ID : 2.

5. Utilisation selon la revendication 3, dans laquelle le polypeptide de l'OPG comprend les résidus d'acides aminés 22 à 194 compris tels que représentés par SEQ ID NO : 2.

6. Utilisation selon l'une quelconque des revendications 3 à 5, dans laquelle le polypeptide de l'OPG est un polypeptide de fusion de l'OPG.

7. Utilisation selon la revendication 6, dans laquelle le polypeptide de fusion de l'OPG comprend une fusion d'une région Fc à l'extrémité N-terminale ou C-terminale du polypeptide de l'OPG.

8. Utilisation selon la revendication 7, dans laquelle le polypeptide de fusion de l'OPG comprend une région Fc fusionnée aux résidus d'acides aminés 22 à 194 de SEQ ID NO : 2.

9. Utilisation selon la revendication 8, dans laquelle le polypeptide de fusion de l'OPG est constitué de la séquence d'acides aminés telle que représentée par SEQ ID NO : 5 ou SEQ ID NO : 8.

10. Utilisation selon la revendication 2, dans laquelle la chimiothérapie comprend des anthracyclines, du taxol, du tamoxifène, de la doxorubicine et du 5-fluorouracile.

11. Utilisation selon la revendication 2, dans laquelle les anticorps se lient à Her2, CDC20, CDC33, une glycoprotéine de type mucine ou au récepteur du facteur de croissance des cellules épidermiques (EGFR) à la surface des cellules tumorales.

12. Utilisation selon la revendication 2, dans laquelle l'agent pour la thérapie d'un cancer comprend un antagoniste de l'hormone de libération de l'hormone lutéinisante (LHRH) de la structure suivante :
A-B-C-D-E-F-G-H-I-J
dans laquelle
A représente pyro-glu, Ac-D-Nal, Ac-D-Qal, Ac-Sar, ou Ac-D-Pal ;
B représente His ou 4-Cl-D-Phe ;
C représente Trp, D-Paf, D-Nal, L-Nal-D-Pal(N-O), ou D-Trp ;
D représente Ser ;
E représente N-Me-Ala, Tyr, N-Me-Tyr, Ser, Lys(iPr), 4-Cl-Phe, His, Asn, Met, Ala, Arg ou Ile ;
F représente 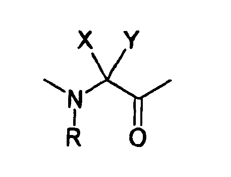 où R et X représentent indépendamment H et un groupe alkyle, et Y comprend une petite entité polaire ;
G représente Leu ou Trp ;
H représente Lys(iPr), Gln, Met, ou Arg ;
I représente Pro ; et
J représente Gly-NH₂ ou D-Ala-NH₂.

13. Utilisation selon la revendication 12, dans laquelle l'antagoniste de la LHRH comprend le peptide : N-Ac-D-Nal-4-Cl-Phe-D-Pal-Ser-N-Me-Tyr-D-Asn-Leu-Lys(iPr)-Pro-D-Ala-NH2.

14. Utilisation selon la revendication 1 ou 7, dans laquelle la quantité thérapeutiquement efficace d'un polypeptide de l'OPG ou d'un polypeptide de fusion de l'OPG est entre 0,1 mg/kg et 10 mg/kg.

15. Utilisation d'un polypeptide de l'OPG pour la préparation d'un médicament destiné à la prévention ou au traitement d'un myélome multiple.
